# EUROPEAN PATENT APPLICATION

(11) **EP 4 721 741 A1**
(43) Date of publication of application: **08.04.2026**
(21) Application number: 24810466.3
(22) Date of filing: 23.05.2024
(51) Int. Cl.: A61K 31/506, A61K 47/10, A61K 9/06, A61K 9/00, A61P 37/02, A61P 19/02, A61P 17/06, A61P 27/02, A61P 17/00

(54) **PHARMACEUTICAL COMPOSITION, TOPICAL PREPARATION, AND PREPARATION METHOD THEREFOR AND USE THEREOF**

(30) Priority: 24.05.2023 WO PCT/CN2023/096089
(71) Applicant: Lynk Pharmaceuticals Co. Ltd., Hangzhou, Zheijiang 310018 (CN)
(72) Inventor: ZHANG, Yuanyuan, Hangzhou Economic & Technological Development Zone Hangzhou, Zhejiang 310018 (CN); GAO, Yujun, Hangzhou Economic & Technological Development Zone Hangzhou, Zhejiang 310018 (CN); XU, Huaiyou, Hangzhou Economic & Technological Development Zone Hangzhou, Zhejiang 310018 (CN); LI, Xiaodong, Hangzhou Economic & Technological Development Zone Hangzhou, Zhejiang 310018 (CN); LV, Lili, Hangzhou Economic & Technological Development Zone Hangzhou, Zhejiang 310018 (CN); LI, Lu, Hangzhou Economic & Technological Development Zone Hangzhou, Zhejiang 310018 (CN); WAN, Zhaokui, Hangzhou Economic & Technological Development Zone Hangzhou, Zhejiang 310018 (CN); VAZQUEZ, Michael Lawrence, Hangzhou Economic & Technological Development Zone Hangzhou, Zhejiang 310018 (CN)
(74) Representative: dompatent
(86) International application number: PCT/CN2024/094984
(87) International publication number: WO 2024/240229

(57) **Abstract**

Disclosed in the present invention are a pharmaceutical composition, a topical preparation, and a preparation method therefor and the use thereof. The pharmaceutical composition of the present invention comprises a pharmaceutically active ingredient, diethylene glycol monoethyl ether and polyethylene glycol 400; and the pharmaceutical composition does not comprise water, wherein the mass ratio of diethylene glycol monoethyl ether to polyethylene glycol 400 is 1:(0.5-4), and the pharmaceutically active ingredient has structural formula (I). The topical preparation prepared from the pharmaceutical composition provided in the present invention has stable properties, will not crystallize out, has a good skin feeling, can promote the rapid permeation of a drug into the stratum corneum, and has a good retention capacity on the epidermis and the dermis, and can thus deliver a drug to the skin in a targeted manner, can reduce systemic side effects and achieve a higher local concentration, has a stronger therapeutic effect and stability, and can reduce safety risks.

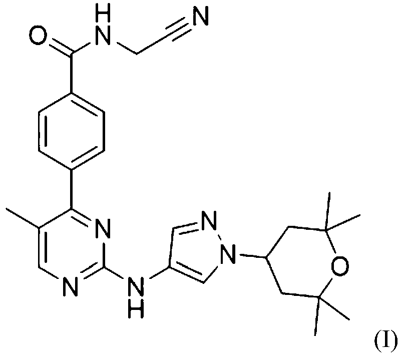

## Description

### SPECIFICATION

The present application claims priority of International Patent Application PCT/CN2023/096089, filed on May 24, 2023. The present application incorporates the entire content of the aforementioned Chinese patent application by reference.

### TECHNICAL FIELD

The present disclosure relates to a pharmaceutical composition, a topical preparation, and a preparation method therefor and a use thereof.

### BACKGROUND

Cytokines of skin immune diseases activate intracellular JAK kinases in various cells through their respective receptors, regulating the differentiation and proliferation of related immune cells as well as the inhibition of cytokine secretion. The JAK/STAT pathway can inhibit the activation of immune cells and T-cell-mediated inflammation, making it a popular target for inflammatory diseases. JAnus kinases belong to the cytoplasmic protein tyrosine family that includes JAK1, JAK2, JAK3, and TYK2.

Currently, there are multiple JAK inhibitors used for the treatment of skin lesions, with oral formulations including: Tofacitinib, Deucravacitinib, Baricitinib, Ruxolitinib, among others. The oral tofacitinib, which completed Phase III clinical trials, has been suspended due to the FDA's requirement for additional safety data, as systemic administration may cause severe adverse reactions. Local administration of active pharmaceutical ingredients has become the preferred delivery method for treating skin conditions such as rheumatoid arthritis, psoriasis, and alopecia areata.

However, the current topical formulations containing active pharmaceutical ingredients still require improvement in skin retention and permeability during application.

### SUMMARY

The technical problem to be solved by the present disclosure is to overcome the drawbacks of low skin retention and poor permeability of topical preparations in the prior art, and thus the present disclosure provides a pharmaceutical composition, a topical preparation, a preparation method therefor, and a use thereof. The topical preparation made from the pharmaceutical composition provided in the present disclosure exhibits stable properties. It does not crystallize, provides favorable skin feel, and can promote rapid permeation of a drug into the stratum corneum. It also demonstrates excellent retention capacity in both the epidermis and dermis, enabling targeted drug delivery to the skin, which reduces systemic side effects while achieving high local concentrations, thereby offering strong therapeutic efficacy, stability, and reduced safety risks.

The present disclosure addresses the above technical problem through the following technical solution.

**The present disclosure provides a pharmaceutical composition,** comprising an active pharmaceutical ingredient, diethylene glycol monoethyl ether, and polyethylene glycol 400 (PEG400), wherein the pharmaceutical composition does not comprise water;
wherein the mass ratio of diethylene glycol monoethyl ether to polyethylene glycol 400 is 1:(0.5 to 4.0); and the active pharmaceutical ingredient has a structure of formula (I):

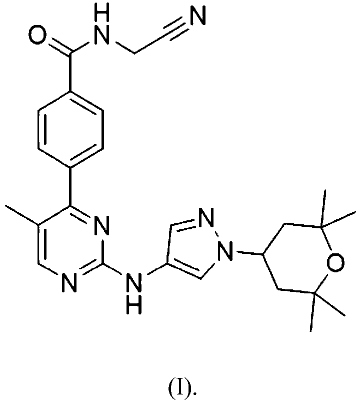

In the present disclosure, the active pharmaceutical ingredient having the structure of formula (I) can be prepared by the preparation method disclosed in Example 113 of CN113227074A.

In the present disclosure, the active pharmaceutical ingredient may be used in an amount of 0.001% to 10%, such as 0.1%, 0.3%, 0.6%, 1.0%, 1.2%, 1.5%, or 2.0%, wherein % refers to the mass percentage of the active pharmaceutical ingredient in the pharmaceutical composition.

In the present disclosure, diethylene glycol monoethyl ether not only functions to enhance or accelerate drug permeation through the skin but also serves as a vehicle for dissolving compounds.

In the present disclosure, diethylene glycol monoethyl ether may be used in an amount of 15% to 40%, such as 20.0%, 23.0%, or 33.8%, wherein % refers to the mass percentage of diethylene glycol monoethyl ether in the pharmaceutical composition.

In the present disclosure, the mass ratio of the active pharmaceutical ingredient to diethylene glycol monoethyl ether is preferably 1:(10.0 to 250.0), such as 1:13.33, 1:15.33, 1:16.67, 1:16.90, 1:20, 1:23, 1:33.33, 1:66.67, 1:76.67, 1:200, or 1:230.

In the present disclosure, polyethylene glycol 400 may be used in an amount of 30% to 55%, such as 30.4%, 38.8%, 45.3%, 52.5%, or 52.9%, wherein % refers to the mass percentage of polyethylene glycol 400 in the pharmaceutical composition.

In the present disclosure, the mass ratio of diethylene glycol monoethyl ether to polyethylene glycol 400 is preferably 1:(0.5 to 3.0), such as 1:0.90, 1:1.94, 1:1.97, 1:2.63, or 1:2.65.

In the present disclosure, preferably, the pharmaceutical composition further comprises polyethylene glycol 3350 (PEG3350).

Herein, when the pharmaceutical composition comprises polyethylene glycol 3350, then polyethylene glycol 3350 may be used in an amount of 25.0% to 50%, preferably 25.0% to 45.0%, such as 25.8%, 26.3%, 26.5%, 27%, 27.2%, 30%, 30.5%, 31.2%, 31.4%, 33.8%, or 40%.

Herein, when the pharmaceutical composition comprises polyethylene glycol 3350, then the mass ratio of diethylene glycol monoethyl ether to polyethylene glycol 3350 may be 1:(0.5 to 3.6), preferably 1:(0.5 to 3.0), such as 1:1.00, 1:1.29, 1:1.30, 1:1.32, 1:1.33, 1:1.35, 1:1.36, 1:1.37, or 1:2.00.

In the present disclosure, preferably, the pharmaceutical composition further comprises one or more of an antioxidant, preservative, hardening agent, and moisturizing agent.

Herein, the antioxidant may be an aromatic amine antioxidant, such as dibutyl hydroxytoluene.

Herein, the antioxidant may be used in an amount of 0.05% to 0.2%, such as 0.1%.

Herein, the preservative may be one or more of methylparaben, sorbic acid, and salts thereof, such as methylparaben.

Herein, the preservative may be used in an amount of 0.05% to 0.2%, such as 0.1%.

Herein, the hardening agent may be one or more of paraffin, beeswax, cetearyl alcohol, white petrolatum, and glyceryl monostearate.

Herein, the hardening agent may be used in an amount of 5% to 40%, such as 9.8%, 22.1%, 30.65%, or 37.6%.

Herein, the moisturizing agent may be one or more of glycerol, light liquid paraffin, soybean oil, medium-chain triglycerides, sodium hyaluronate, urea, propylene glycol, butylene glycol, panthenol, sodium pyrrolidone carboxylate, and trehalose, preferably propylene glycol.

Herein, the moisturizing agent may be used in an amount of 2% to 80%, such as 5.0%, 9.0%, 20.0%, 37.5%, or 77.5%.

In the present disclosure, the pharmaceutical composition may be in the form of a solution, gel, ointment, or cream.

In the present disclosure, when the pharmaceutical composition is a semi-solid, such as a cream, then the active pharmaceutical ingredient may have a particle size of less than 180 µm.

In the present disclosure, the components in the pharmaceutical composition, after mixing, exhibit no crystallization and form a single-phase system.

In some preferred embodiments, the pharmaceutical composition comprises the aforementioned active pharmaceutical ingredient, diethylene glycol monoethyl ether, polyethylene glycol 400, and polyethylene glycol 3350, wherein the pharmaceutical composition does not comprise water;
wherein the mass ratio of diethylene glycol monoethyl ether to polyethylene glycol 400 is 1:(0.5 to 4.0), and the mass ratio of diethylene glycol monoethyl ether to polyethylene glycol 3350 is 1:(0.5 to 3.6).

In some preferred embodiments, the pharmaceutical composition comprises the aforementioned active pharmaceutical ingredient, diethylene glycol monoethyl ether, polyethylene glycol 400, and polyethylene glycol 3350, wherein the pharmaceutical composition does not comprise water;
wherein the mass ratio of diethylene glycol monoethyl ether to polyethylene glycol 400 is 1:(0.5 to 3.0), and the mass ratio of diethylene glycol monoethyl ether to polyethylene glycol 3350 is 1:(0.5 to 3.0).

In some preferred embodiments, the pharmaceutical composition comprises, in weight percent,

| | |
|---|---|
| active pharmaceutical ingredient | 0.1% to 2.0% |
| diethylene glycol monoethyl ether | 20.0% to 33.8% |
| polyethylene glycol 400 | 30.4% to 52.9% |
| polyethylene glycol 3350 | 25.8% to 40.0%. |

In some preferred embodiments, the pharmaceutical composition comprises, in weight percent,

| | |
|---|---|
| active pharmaceutical ingredient | 0.1% to 2.0% |
| diethylene glycol monoethyl ether | 20.0% to 33.8% |
| polyethylene glycol 400 | 30.4% to 52.9% |
| polyethylene glycol 3350 | 25.8% to 40.0% |
| butylated hydroxytoluene | 0% to 0.1% |
| methylparaben | 0% to 0.1% |
| tartrazine | 0% to 0.001% |
| beeswax | 0% to 30.65% |
| cetearyl alcohol | 0% to 9.8% |
| white petrolatum | 0% to 22.1% |
| propylene glycol | 0% to 20.0%. |

**The present disclosure further provides a topical preparation,** comprising the aforementioned pharmaceutical composition.

In the present disclosure, the topical preparation may be a liquid preparation or a semi-solid preparation.

Herein, the liquid preparation may be a liniment or tincture.

Herein, the semi-solid preparation may be a cream, organogel, ointment, or paste.

In some preferred embodiments, the ointment is formulated to comprise, in weight percent,

| | |
|---|---|
| active pharmaceutical ingredient | 0.1% to 2.0% |
| diethylene glycol monoethyl ether | 20.0% to 33.8% |
| polyethylene glycol 400 | 30.4% to 52.9% |
| polyethylene glycol 3350 | 25.8% to 40.0%. |

Preferably, the ointment is formulated to comprise, in weight percent,

| | |
|---|---|
| active pharmaceutical ingredient | 0.6% to 2.0% |
| diethylene glycol monoethyl ether | 20.0% to 33.8% |
| polyethylene glycol 400 | 30.4% to 52.9% |
| polyethylene glycol 3350 | 26.3% to 40.0%. |

More preferably, the ointment is formulated to comprise, in weight percent,

| | |
|---|---|
| active pharmaceutical ingredient | 0.6% |
| diethylene glycol monoethyl ether | 20.0% |
| polyethylene glycol 400 | 52.9% |
| polyethylene glycol 3350 | 26.5%. |

More preferably, the ointment is formulated to comprise, in weight percent,

| | |
|---|---|
| active pharmaceutical ingredient | 1.2% |
| diethylene glycol monoethyl ether | 20.0% |
| polyethylene glycol 400 | 52.5% |
| polyethylene glycol 3350 | 26.3%. |

More preferably, the ointment is formulated to comprise, in weight percent,

| | |
|---|---|
| active pharmaceutical ingredient | 1.2% |
| diethylene glycol monoethyl ether | 20.0% |
| polyethylene glycol 400 | 38.8% |
| polyethylene glycol 3350 | 40.0%. |

More preferably, the ointment is formulated to comprise, in weight percent,

| | |
|---|---|
| active pharmaceutical ingredient | 2.0% |
| diethylene glycol monoethyl ether | 33.8% |
| polyethylene glycol 400 | 30.4% |
| polyethylene glycol 3350 | 33.8%. |

Preferably, the ointment is formulated to comprise, in weight percent,

| | |
|---|---|
| active pharmaceutical ingredient | 0.1% to 1.5% |
| diethylene glycol monoethyl ether | 20.0% |
| polyethylene glycol 400 | 52.5% |
| polyethylene glycol 3350 | 25.8-27.2% |
| butylated hydroxytoluene | 0.1% |
| methylparaben | 0.1%. |

More preferably, the ointment is formulated to comprise, in weight percent,

| | |
|---|---|
| active pharmaceutical ingredient | 0.1% |
| diethylene glycol monoethyl ether | 20.0% |
| polyethylene glycol 400 | 52.5% |
| polyethylene glycol 3350 | 27.2% |
| butylated hydroxytoluene | 0.1% |
| methylparaben | 0.1%. |

More preferably, the ointment is formulated to comprise, in weight percent,

| | |
|---|---|
| active pharmaceutical ingredient | 0.3% |
| diethylene glycol monoethyl ether | 20.0% |
| polyethylene glycol 400 | 52.5% |
| polyethylene glycol 3350 | 27.0% |
| butylated hydroxytoluene | 0.1% |
| methylparaben | 0.1%. |

More preferably, the ointment is formulated to comprise, in weight percent,

| | |
|---|---|
| active pharmaceutical ingredient | 1.0% |
| diethylene glycol monoethyl ether | 20.0% |
| polyethylene glycol 400 | 52.5% |
| polyethylene glycol 3350 | 26.3% |
| butylated hydroxytoluene | 0.1% |
| methylparaben | 0.1%. |

More preferably, the ointment is formulated to comprise, in weight percent,

| | |
|---|---|
| active pharmaceutical ingredient | 1.5% |
| diethylene glycol monoethyl ether | 20.0% |
| polyethylene glycol 400 | 52.5% |
| polyethylene glycol 3350 | 25.8% |
| butylated hydroxytoluene | 0.1% |
| methylparaben | 0.1%. |

Preferably, the ointment is formulated to comprise, in weight percent,

| | |
|---|---|
| active pharmaceutical ingredient | 0.1% to 1.5% |
| diethylene glycol monoethyl ether | 23.0% |
| polyethylene glycol 400 | 45.3% |
| polyethylene glycol 3350 | 30.0% to 31.4% |
| butylated hydroxytoluene | 0.1% |
| methylparaben | 0.1% |
| tartrazine | 0.00025% to 0.001%. |

More preferably, the ointment is formulated to comprise, in weight percent,

| | |
|---|---|
| active pharmaceutical ingredient | 0.1% |
| diethylene glycol monoethyl ether | 23.0% |
| polyethylene glycol 400 | 45.3% |
| polyethylene glycol 3350 | 31.4% |
| butylated hydroxytoluene | 0.1% |
| methylparaben | 0.1% |
| tartrazine | 0.001%. |

More preferably, the ointment is formulated to comprise, in weight percent,

| | |
|---|---|
| active pharmaceutical ingredient | 0.3% |
| diethylene glycol monoethyl ether | 23.0% |
| polyethylene glycol 400 | 45.3% |
| polyethylene glycol 3350 | 31.2% |
| butylated hydroxytoluene | 0.1% |
| methylparaben | 0.1% |
| tartrazine | 0.0007%. |

More preferably, the ointment is formulated to comprise, in weight percent,

| | |
|---|---|
| active pharmaceutical ingredient | 1.0% |
| diethylene glycol monoethyl ether | 23.0% |
| polyethylene glycol 400 | 45.3% |
| polyethylene glycol 3350 | 30.5% |
| butylated hydroxytoluene | 0.1% |
| methylparaben | 0.1% |
| tartrazine | 0.00025%. |

More preferably, the ointment is formulated to comprise, in weight percent,

| | |
|---|---|
| active pharmaceutical ingredient | 1.5% |
| diethylene glycol monoethyl ether | 23.0% |
| polyethylene glycol 400 | 45.3% |
| polyethylene glycol 3350 | 30.0% |
| butylated hydroxytoluene | 0.1% |
| methylparaben | 0.1%. |

Preferably, the ointment comprises, in weight percent,

| | |
|---|---|
| active pharmaceutical ingredient | 0.1 to 2.0% |
| diethylene glycol monoethyl ether | 20.0 to 33.8% |
| polyethylene glycol 400 | 30.4% to 52.9% |
| polyethylene glycol 3350 | 25.8% to 50.0% |
| paraffin | 0% to 5% |
| beeswax | 0% to 30.65% |
| cetearyl alcohol | 0% to 9.8% |
| white petrolatum | 0% to 22.6% |
| glyceryl monostearate | 0% to 10% |
| propylene glycol | 0% to 20.0% |
| butylated hydroxytoluene | 0% to 0.1% |
| methylparaben | 0% to 0.1% |
| polyethylene glycol 600 | 0% to 25.0% |
| light liquid paraffin | 0% to 5.0% |
| soybean oil | 0% to 5.0% |
| medium-chain triglycerides | 0% to 5.0%. |

Preferably, the ointment comprises, in weight percent,

| | |
|---|---|
| active pharmaceutical ingredient | 0.1% to 2.0% |
| diethylene glycol monoethyl ether | 20.0% to 33.8% |
| polyethylene glycol 400 | 30.4% to 52.9% |
| polyethylene glycol 3350 | 25.8% to 40.0% |
| butylated hydroxytoluene | 0% to 0.1% |
| methylparaben | 0% to 0.1% |
| tartrazine | 0% to 0.001% |
| beeswax | 0% to 30.65% |
| cetearyl alcohol | 0% to 9.8% |
| white petrolatum | 0% to 22.1% |
| propylene glycol | 0% to 20.0%. |

**The present disclosure further provides a method for preparing the aforementioned pharmaceutical composition,** comprising the following steps:
(1) preparing an API solution by mixing an active pharmaceutical ingredient, diethylene glycol monoethyl ether, and polyethylene glycol 400 at 50°C to 80°C until dissolved;
(2) preparing a matrix solution by heating polyethylene glycol 3350 to melting at 50°C to 80°C;
(3) preparing an ointment by mixing the API solution and the matrix solution with stirring.

In step (1), a temperature at which the mixing is performed is preferably 50°C or 80°C.

In step (2), preferably, raw materials further comprise one or more of an antioxidant, preservative, hardening agent, and moisturizing agent.

In step (2), preferably, raw materials further comprise diethylene glycol monoethyl ether.

Herein, preferably, when the raw materials in step (2) comprise diethylene glycol monoethyl ether, then diethylene glycol monoethyl ether in step (1) is used in an amount that is 2/3 of the prescribed amount, and diethylene glycol monoethyl ether in step (2) is used in an amount that is 1/3 of the prescribed amount.

In a preferred embodiment, the preparation of the matrix solution comprises the following steps: adding methylparaben to diethylene glycol monoethyl ether and stirring at 50°C to 80°C until completely dissolved; then adding butylated hydroxytoluene and polyethylene glycol 3350 and stirring until melted.

In step (2), a temperature at which the heating is performed is preferably 50°C or 80°C.

In step (3), a speed at which the stirring is performed may be conventional in the art, preferably 315 rpm.

In step (3), a duration for which the stirring is performed may be conventional in the art, preferably 10 min to 15 min.

In step (3), a temperature at which the mixing is performed is preferably 50°C to 60°C.

After the mixing in step (3), a cooling operation may further be included.

Herein, the cooling may be performed by means of circulating water cooling.

**The present disclosure further provides a use of the aforementioned pharmaceutical composition or topical preparation in the preparation of a medicament for treating an autoimmune disease.**

In the present disclosure, the autoimmune disease may be arthritis, rheumatoid arthritis, psoriasis, psoriasis, osteoarthritis, regional enteritis, ankylosing spondylitis, autoimmune eye disease, dry eye syndrome, atopic dermatitis, contact dermatitis, systemic lupus erythematosus, or alopecia areata.

The present disclosure further provides a method for treating an autoimmune disease, comprising administering to a subject a therapeutically effective amount of the aforementioned pharmaceutical composition or topical preparation.

In the present disclosure, the autoimmune disease may be arthritis, rheumatoid arthritis, psoriasis, psoriasis, osteoarthritis, regional enteritis, ankylosing spondylitis, autoimmune eye disease, dry eye syndrome, atopic dermatitis, contact dermatitis, systemic lupus erythematosus, or alopecia areata.

Based on the common knowledge in the field, the above preferred conditions can be combined arbitrarily to obtain various preferred embodiments of the present disclosure.

In the present disclosure, except for the active pharmaceutical ingredient, all reagents and raw materials used are commercially available.

The positive progressive effect of the present disclosure is that:

The topical preparation made from the pharmaceutical composition provided in the present disclosure exhibits stable properties. It does not crystallize, provides favorable skin feel, and can promote rapid permeation of a drug into the stratum corneum. It also demonstrates excellent retention capacity in both the epidermis and dermis, enabling targeted drug delivery to the skin, which reduces systemic side effects while achieving high local concentrations, thereby offering strong therapeutic efficacy, stability, and reduced safety risks.

### BRIEF DESCRIPTION OF THE DRAWINGS

FIG. 1 shows the microscopic observation results of the ointment prepared in 3A of Example 3. In this context, part (a) of FIG. 1 represents the initial condition, while part (b) of FIG. 1 depicts the accelerated condition after 6 months of storage.
FIG. 2 shows the microscopic observation results of the cream prepared in Example 7 under initial conditions.
FIG. 3 shows the microscopic observation results of the hydrogel prepared in Example 10 under initial conditions.
FIG. 4 shows the microscopic observation results of the organogel prepared in Example 12 under initial conditions. In this context, part (a) of FIG. 4 represents the initial condition, while part (b) of FIG. 4 depicts the accelerated condition after 6 months of storage.
FIG. 5 shows the microscopic observation results of the liniment prepared in Example 13.
FIG. 6 is a curve showing the cumulative drug release amount per unit area versus the square root of time in Effect Example 2.
FIG. 7 is a curve showing the release rate versus specification in Effect Example 2.

### DETAILED DESCRIPTION OF THE PREFERRED EMBODIMENT

The following examples further illustrate the present disclosure but do not limit the present disclosure to the scope of the described examples. In the following examples, experimental methods without specified conditions were carried out according to conventional methods and conditions, or selected as per commercial instructions.

In the specific embodiments of the present disclosure, the active pharmaceutical ingredient having the structure of formula (I) is prepared using the preparation method disclosed in Example 113 of CN113227074A.

### Example 1: Ointment

The ointment formulation is shown in Table 1 below.

**Table 1**

| **No.** | **1A** | **1B** | **1C** | **1D** | **Role** |
|---|---|---|---|---|---|
| Active pharmaceutical ingredient / % | 0.6 | 1.2 | 1.2 | 2.0 | API |
| Diethylene glycol monoethyl ether / % | 20.0 | 20.0 | 20.0 | 33.8 | Permeation enhancer, vehicle |
| Polyethylene glycol 400 / % | 52.9 | 52.5 | 38.8 | 30.4 | Permeation enhancer, vehicle |
| polyethylene glycol 3350 / % | 26.5 | 26.3 | 40.0 | 33.8 | Ointment matrix |
| Total / % | 100.0 | 100.0 | 100.0 | 100.0 | - |

The preparation process is as follows:
(1) Preparation of an API solution: Prescribed amounts of active pharmaceutical ingredient, diethylene glycol monoethyl ether (Transcutol P), and polyethylene glycol 400 (PEG 400) were weighed into a container and heated in a water bath at 80°C with stirring until complete dissolution.
(2) Preparation of a matrix solution: A prescribed amount of polyethylene glycol 3350 (PEG 3350) was weighed and heated in a water bath at 80°C with stirring until melted.
(3) Preparation of ointment: The melted matrix solution was slowly added to the API solution with stirring. After the addition, the mixture was stirred for 10 minutes at a stirring speed of 315 rpm, and cooled to form the ointment.

### Example 2: Ointment

The ointment formulation is shown in Table 2 below.

**Table 2**

| **No.** | **2A** | **2B** | **2C** | **2D** | **Role** |
|---|---|---|---|---|---|
| Active pharmaceutical ingredient / % | 0.1 | 0.3 | 1.0 | 1.5 | API |
| Diethylene glycol monoethyl ether / % | 20.0 | 20.0 | 20.0 | 20.0 | Permeation enhancer, vehicle |
| Polyethylene glycol 400 / % | 52.5 | 52.5 | 52.5 | 52.5 | Permeation enhancer, vehicle |
| Polyethylene glycol 3350 / % | 27.2 | 27.0 | 26.3 | 25.8 | Ointment matrix |
| Butylated hydroxytoluene / % | 0.1 | 0.1 | 0.1 | 0.1 | Antioxidant |
| Methylparaben / % | 0.1 | 0.1 | 0.1 | 0.1 | Preservative |
| Total / % | 100.0 | 100.0 | 100.0 | 100.0 | - |

The preparation process is as follows:
(1) Preparation of an API solution: Prescribed amounts of active pharmaceutical ingredient, diethylene glycol monoethyl ether (Transcutol P), and polyethylene glycol 400 (PEG 400) were weighed into a container and heated in a water bath at 80°C with stirring until the active pharmaceutical ingredient was completely dissolved.
(2) Preparation of a matrix solution: A prescribed amount of polyethylene glycol 3350 (PEG 3350) was weighed and heated in a water bath at 80°C with stirring until melted.
(3) Preparation of ointment: The melted matrix solution was slowly added to the API solution with stirring. After the addition, the mixture was stirred at a stirring speed of 310 rpm and cooled to 35°C, then allowed to cool to room temperature.

### Example 3: Ointment

The ointment formulation is shown in Table 3 below.

**Table 3**

| **No.** | **3A** | **3B** | **3C** | **3D** | **Role** |
|---|---|---|---|---|---|
| Active pharmaceutical | 0.1 | 0.3 | 1.0 | 1.5 | API |
| ingredient / % | | | | | |
| Diethylene glycol monoethyl ether / % | 23.0 | 23.0 | 23.0 | 23.0 | Permeation enhancer, vehicle |
| Polyethylene glycol 400 / % | 45.3 | 45.3 | 45.3 | 45.3 | Permeation enhancer, vehicle |
| Polyethylene glycol 3350 / % | 31.399 | 31.1993 | 30.49975 | 30.0 | Ointment matrix |
| Butylated hydroxytoluene / % | 0.1 | 0.1 | 0.1 | 0.1 | Antioxidant |
| Methylparaben / % | 0.1 | 0.1 | 0.1 | 0.1 | Preservative |
| Tartrazine / % | 0.001 | 0.0007 | 0.00025 | 0 | Pigment |
| Total / % | 100.0 | 100.0 | 100.0 | 100.0 | - |

The preparation process is as follows:
(1) Preparation of an API solution: A prescribed amount of active pharmaceutical ingredient and diethylene glycol monoethyl ether (Transcutol P), and two-thirds of the prescribed amount of polyethylene glycol 400 (PEG 400) were weighed into a container and heated in a water bath at 50°C to 60°C with stirring until completely dissolved.
(2) Preparation of a matrix solution: A prescribed amount of methylparaben was weighed and added to one-third of the prescribed amount of PEG400, with stirring at 70±5°C to completely dissolve the methylparaben. Then the prescribed amounts of polyethylene glycol 3350 (PEG 3350) and butylated hydroxytoluene were added and stirred at 70±5°C until PEG 3350 and butylated hydroxytoluene were fully melted. The mixture was cooled down to 50°C to 60°C.
(3) Preparation of ointment: While maintaining a temperature of 50°C to 60°C, the API solution was poured into the matrix solution with continuous stirring. After stirring for 15 minutes, tartrazine was added to adjust the color, and the mixture was further stirred and cooled to room temperature.

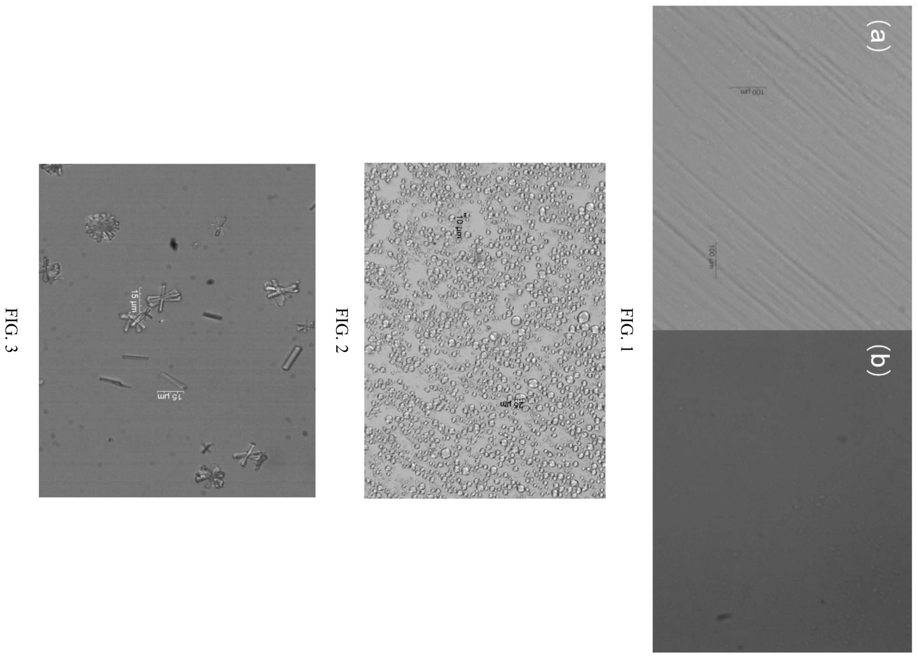

A clean glass slide was taken and an appropriate amount of sample 3A (approximately 0.01g per sampling) was applied onto the slide, which was covered with a coverslip. The coverslip was gently pressed. The ointment was observed under a microscope with an eyepiece magnification of 10X, an objective magnification of 40X, and polarized light at 300° (under initial conditions and accelerated conditions at 40°C, 75% RH for 6 months). The results, as shown in parts (a) and (b) of FIG. 1, indicate no crystallization in the ointment.

### Example 4: Ointment

The ointment formulation is shown in Table 4 below.

**Table 4**

| **No.** | **4A** | **4B** | **4C** | **4D** | **Role** |
|---|---|---|---|---|---|
| Active pharmaceutical ingredient / % | 0.8 | 1.1 | 2.0 | 1.6 | API |
| Diethylene glycol monoethyl ether / % | 14.5 | 20.1 | 20.0 | 29.0 | Permeation enhancer, vehicle |
| Polyethylene glycol 400 / % | 12.5 | 17.34 | 24.0 | / | Permeation enhancer, vehicle |
| Paraffin / % | / | / | / | 5.0 | Hardening agent |
| Beeswax / % | / | 30.65 | / | / | Hardening agent |
| Cetearyl alcohol / % | / | / | 9.8 | / | Hardening agent |
| White petrolatum / % | 22.1 | / | / | 22.6 | Hardening agent |
| Glyceryl monostearate / % | / | / | / | 10.0 | Hardening agent |
| Propylene glycol / % | / | / | 20.0 | 9.0 | Vehicle, moisturizing agent |
| Polyethylene glycol 3350 / % | 50.0 | 30.65 | 24.0 | 22.3 | Ointment matrix |
| Butylated hydroxytoluene / % | 0.05 | 0.07 | 0.1 | 0.1 | Antioxidant |
| Methylparaben / % | 0.05 | 0.09 | 0.1 | 0.1 | Preservative |
| Total / % | 100.0 | 100.0 | 100.0 | 100.0 | - |

The preparation process is as follows:
(1) Preparation of an API solution: Prescribed amounts of active pharmaceutical ingredient, diethylene glycol monoethyl ether (Transcutol P), and polyethylene glycol 400 (PEG 400) were weighed into a container and heated in a water bath at 80°C with stirring until complete dissolution.
(2) Preparation of a matrix solution: Prescribed amounts of polyethylene glycol 3350 (PEG 3350), a hardening agent, butylated hydroxytoluene, methylparaben, and propylene glycol were weighed and heated in a water bath at 80°C with stirring until melted.
(3) Preparation of ointment: The melted matrix solution was slowly added to the API solution with stirring. After the addition, the mixture was stirred for 10 minutes at a stirring speed of 315 rpm, then allowed to cool to room temperature.

### Example 5: Ointment

The ointment formulation is shown in Table 5 below.

**Table 5**

| **No.** | **5A** | **5B** | **5C** | **5D** | **Role** |
|---|---|---|---|---|---|
| Active pharmaceutical ingredient / % | 1.5 | 1.5 | 1.5 | 1.5 | API |
| Diethylene glycol monoethyl ether / % | 23.0 | 20.0 | 20.0 | 20.0 | Permeation enhancer, vehicle |
| Polyethylene glycol 400 / % | 45.5 | 48.5 | 43.5 | 52.7 | Permeation enhancer, vehicle |
| Polyethylene glycol 3350 / % | 30.0 | 30.0 | 35.0 | 25.8 | Ointment matrix |
| Total / % | 100.0 | 100.0 | 100.0 | 100.0 | - |

The preparation process is as follows:
(1) Preparation of an API solution: Prescribed amounts of active pharmaceutical ingredient, diethylene glycol monoethyl ether (Transcutol P), and polyethylene glycol 400 (PEG 400) were weighed into a container and heated in a water bath at 80°C with stirring until complete dissolution.
(2) Preparation of a matrix solution: A prescribed amount of polyethylene glycol 3350 (PEG 3350) was weighed and heated in a water bath at 80°C with stirring until melted.
(3) Preparation of ointment: The melted matrix solution was slowly added to the API solution with stirring. After the addition, the mixture was stirred for 10 minutes at a stirring speed of 315 rpm, then allowed to cool to room temperature.

### Example 6: Ointment

The ointment formulation is shown in Table 6 below.

**Table 6**

| **No.** | **6A** | **6B** | **6C** | **6D** | **Role** |
|---|---|---|---|---|---|
| Active pharmaceutical ingredient / % | 1.6 | 1.6 | 1.6 | 1.6 | API |
| Diethylene glycol monoethyl ether / % | 29.0 | 29.0 | 29.0 | 29.0 | Permeation enhancer, vehicle |
| Polyethylene glycol 600 / % | 25.0 | 25.0 | 25.0 | 25.0 | Permeation enhancer, vehicle |
| Propylene glycol / % | 5.0 | / | / | / | Moisturizing agent, vehicle |
| Light liquid paraffin / % | / | 5.0 | / | / | Moisturizing agent |
| Soybean oil / % | / | / | 5.0 | / | Moisturizing agent |
| Medium-chain triglycerides / % | / | / | / | 5.0 | Moisturizing agent |
| Polyethylene glycol 3350 / % | 39.2 | 39.2 | 39.2 | 39.2 | Ointment matrix |
| Butylated hydroxytoluene / % | 0.1 | 0.1 | 0.1 | 0.1 | Antioxidant |
| Methylparaben / % | 0.1 | 0.1 | 0.1 | 0.1 | Preservative |
| Total / % | 100.0 | 100.0 | 100.0 | 100.0 | / |

The preparation process is as follows:
(1) Preparation of an API solution: Prescribed amounts of active pharmaceutical ingredient, diethylene glycol monoethyl ether (Transcutol P), polyethylene glycol 600 (PEG 600), and moisturizing agent were weighed into a container and heated in a water bath at 80°C with stirring until complete dissolution.
(2) Preparation of a matrix solution: A prescribed amount of polyethylene glycol 3350 (PEG 3350) was weighed and heated in a water bath at 80°C with stirring until melted.
(3) Preparation of ointment: The melted matrix solution was slowly added to the API solution with stirring. After the addition, the mixture was stirred for 10 minutes at a stirring speed of 315 rpm, and cooled to form the ointment.

### Example 7: Cream

The cream formulation is shown in Table 7 below.

**Table 7**

| **No.** | **7A** | **Role** |
|---|---|---|
| Active pharmaceutical ingredient / % | 0.6 | API |
| Diethylene glycol monoethyl ether / % | 10.0 | Permeation enhancer, vehicle |
| Propylene glycol / % | 2.00 | Permeation enhancer, vehicle |
| Pegoxol-7 stearate / % | 8.00 | Emulsifier |
| Polyoxyethylene oleate glyceride / % | 2.00 | Emulsifier |
| Glyceryl monostearate / % | 1.5 | Oil phase matrix, thickener |
| Medium-chain triglycerides / % | 5.00 | Oil phase matrix |
| Purified water / % | 70.9 | Aqueous phase matrix |
| Total / % | 100.0 | - |

The preparation process is as follows:
(1) Preparation of an API solution: A prescribed amount of active pharmaceutical ingredient was added to diethylene glycol monoethyl ether (Transcutol P) and heated to 80°C to dissolve the active pharmaceutical ingredient. A prescribed amount of propylene glycol was further added and the mixture was stirred for 3 minutes to achieve uniform mixing.
(2) Preparation of an oil phase: Prescribed amounts of pegoxol-7 stearate, polyoxyethylene oleate glyceride, glyceryl monostearate, and medium-chain triglycerides were weighed and heated at 80°C until melted.
(3) Preparation of a cream: With stirring, the API solution was added to the pre-prepared oil phase and homogenization (at 15,000 rpm) was initiated. The API-containing oil phase was slowly added into purified water at 80°C, followed by homogenization for 10 minutes.

The obtained cream has a uniform texture. Sample 7A was observed under initial conditions according to the method described in Example 3, and the results are shown in FIG. 2. Results indicate that the cream exhibits uniform emulsion droplets with a particle size of approximately 10 to 30 µm.

### Example 8: Cream

The cream formulation is shown in Table 8 below.

**Table 8**

| **No.** | **8A** | **8B** | **8C** | **8D** | **Role** |
|---|---|---|---|---|---|
| Active pharmaceutical ingredient / % | 0.2 | 0.2 | 0.1 | 0.2 | API |
| Diethylene glycol monoethyl ether / % | 10.0 | 10.0 | 10.0 | 10.0 | Permeation enhancer, vehicle |
| Polyethylene glycol 400 / % | 8.0 | 8.0 | 8.0 | 8.0 | Permeation enhancer, vehicle |
| Pegoxol-7 stearate / % | 10.0 | 10.0 | 10.0 | 10.0 | Emulsifier |
| Polyoxyethylene oleate glyceride / % | 4.0 | 4.0 | 4.0 | 4.0 | Emulsifier |
| Glyceryl monostearate / % | 3.0 | 3.0 | 3.0 | 3.0 | Oil phase matrix, thickener |
| Medium-chain triglycerides / % | 5.0 | 5.0 | 5.0 | 5.0 | Oil phase matrix |
| Hypromellose / % | 3.0 | 5.0 | 3.0 | / | Aqueous phase matrix |
| Carbomer / % | / | / | / | 0.2 | Aqueous phase matrix |
| Purified water / % | 56.8 | 54.8 | 56.9 | 59.6 | Aqueous phase matrix |
| Total / % | 100.0 | 100.0 | 100.0 | 100.0 | - |

The preparation process is as follows:
(1) Preparation of an API solution: A prescribed amount of active pharmaceutical ingredient was dissolved in a mixture of diethylene glycol monoethyl ether (Transcutol P) and polyethylene glycol 400 (PEG 400) at 80°C.
(2) Preparation of an oil phase: Prescribed amounts of pegoxol-7 stearate, polyoxyethylene oleate glyceride, glyceryl monostearate, and medium-chain triglycerides were weighed and heated at 80°C until melted.
(3) Preparation of an aqueous phase:
   Hypromellose solution(8A, 8B, 8C): A prescribed amount of purified water was weighed and hypromellose was slowly added with stirring. The mixture was stirred until the hydroxypropyl methylcellulose is completely dissolved.
   Carbomer solution (8D): A prescribed amount of carbomer was dissolved thoroughly in purified water.
(4) Preparation of cream:
   a. Preparation of cream (batch 8A/8B): The oil phase and the API solution were mixed with magnetic stirring in an 80°C water bath for 5 minutes. The mixture was cooled to 50°C and the aqueous phase (hypromellose solution) was added. The mixture was stirred vigorously for 5 minutes (stirring speed: 520 rpm) and then allow to stand for cooling.
   b. Preparation of cream (batch 8C): The oil phase and the API solution were mixed under magnetic stirring in an 80°C water bath for 10 minutes. The mixture was cooled to 50°C and the aqueous phase (hypromellose solution) was added. Homogenization was initiated (homogenization speed 9.0±0.2 Kr/min) and continued for 5 minutes. Homogenization was continued while cooling to 30°C (homogenization speed 8.8 Kr/min).
   c. Preparation of cream (batch 8D): The oil phase and the API solution were mixed in an 80°C water bath with magnetic stirring for 10 minutes. Then the mixture was added to the aqueous phase (Carbomer solution) and stirred vigorously for 5 minutes (360 rpm). Stirring (200 rpm) was continued while cooling to 30°C.

### Example 9: Cream

The cream formulation is shown in Table 9 below.

**Table 9**

| **No.** | **9A** | **9B** | **9C** | **Role** |
|---|---|---|---|---|
| Active pharmaceutical ingredient / % | 1.5 | 1.5 | 1.5 | API |
| Diethylene glycol monoethyl ether / % | 30.0 | 30.0 | 30.0 | Permeation enhancer, vehicle |
| Polyethylene glycol 400 / % | 15.0 | 15.0 | 15.0 | Permeation enhancer, vehicle |
| Pegoxol-7 stearate / % | 12.0 | 12.0 | 12.0 | Emulsifier |
| Cetyl alcohol / % | 3.0 | / | / | Oil phase matrix, thickener |
| Monooleoglyceride / % | / | 3.0 | / | Oil phase matrix, thickener |
| Glyceryl monostearate / % | / | / | 3.0 | Oil phase matrix, thickener |
| Light liquid paraffin / % | 2.0 | 2.0 | 2.0 | Oil phase matrix, thickener |
| Medium-chain triglycerides / % | 6.0 | 6.0 | 6.0 | Oil phase matrix |
| Tween 80 / % | 1.0 | 1.0 | 1.0 | Surfactant |
| Ethylparaben / % | 0.1 | 0.1 | 0.1 | Preservative |
| Purified water / % | 29.4 | 29.4 | 29.4 | Aqueous phase matrix |
| Total / % | 100.0 | 100.0 | 100.0 | - |

The preparation process is as follows:
(1) Preparation of an API solution: A prescribed amount of active pharmaceutical ingredient was dissolved in a mixture of diethylene glycol monoethyl ether (Transcutol P) and polyethylene glycol 400 (PEG 400).
(2) Preparation of an oil phase: Prescribed amounts of pegoxol-7 stearate, cetyl alcohol (9A), monooleoglyceride (9B), glyceryl monostearate (9C), light liquid paraffin, and medium-chain triglycerides were weighed and heated in an 80°C water bath to melt into a liquid mixture.
(3) Preparation of an aqueous phase: Prescribed amounts of Tween 80 and ethylparaben were dissolved in purified water at 70°C.
(4) Preparation of cream: The oil phase and API solution were mixed with magnetic stirring in a 70°C water bath for 5 minutes. The aqueous phase was added at 70°C and subjected to high-speed shearing for 5 minutes. Then an appropriate amount was taken and placed in an EP tube for cooling.

### Example 10: Hydrogel

The hydrogel formulation is shown in Table 10 below.

**Table 10**

| **No.** | **10A** | **Role** |
|---|---|---|
| Active pharmaceutical ingredient / % | 0.2 | API |
| Diethylene glycol monoethyl ether / % | 3.3 | Permeation enhancer, vehicle |
| Polyethylene glycol 400 / % | 2.7 | Permeation enhancer, vehicle |
| Carbomer / % | 1.0 | Gel matrix |
| Purified water / % | 92.8 | Gel matrix |
| 2M sodium hydroxide solution / % | q.s. | pH adjuster |
| Total / % | 100.0 | - |

| | | |
|---|---|---|
| Note: "q.s." in the table denotes a trace amount. | | |

The preparation process is as follows:
(1) Preparation of an API solution: A prescribed amount of active pharmaceutical ingredient was added to a mixture of diethylene glycol monoethyl ether (Transcutol P) and polyethylene glycol 400 (PEG 400). The mixture was stirred at 80°C for 10 minutes to dissolve the active pharmaceutical ingredient.
(2) Preparation of a carbomer solution: A prescribed amount of carbomer was weighed, which was allowed to fully swell using purified water.
(3) Preparation of gel: The API solution was added to the prepared carbomer solution with stirring (stirring speed 205 rpm). A trace amount of 2M sodium hydroxide solution was added. After complete addition, the mixture was stirred for additional 3 minutes, then brought to its final volume with the remaining quantity of water and stirred for 5 minutes (stirring speed 900 rpm).

Sample 10A was observed under a microscope under initial conditions according to the method described in Example 3 and the results are shown in FIG. 3. The results indicate that under initial conditions, the hydrogel exhibited a small amount of crystallization, with a crystal particle size of approximately 15 µm.

### Example 11: Hydrogel

The hydrogel formulation is shown in Table 11 below.

**Table 11**

| **No.** | **11A** | **11B** | **11C** | **11D** | **Role** |
|---|---|---|---|---|---|
| Active pharmaceutical ingredient / % | 2.0 | 1.5 | 1.5 | 1.3 | API |
| Diethylene glycol monoethyl ether / % | 13.5 | 5.0 | / | 21.0 | Permeation enhancer, vehicle |
| Dimethyl sulfoxide / % | / | / | 5.0 | / | Permeation enhancer, vehicle |
| Polyethylene glycol 400 / % | 26.8 | 30.0 | 30.0 | 33.6 | Permeation enhancer, vehicle |
| Carbomer / % | 9.8 | 1.0 | 1.0 | 0.5 | Gel matrix |
| Purified water / % | 42.9 | 57.5 | 57.5 | 38.6 | Gel matrix |
| Triethanolamine / % | Trace amount | Trace amount | Trace amount | Trace amount | Surfactant, pH adjuster |
| Tween 80 / % | 5.0 | 5.0 | 5.0 | 5.0 | Surfactant |
| Total / % | 100 | 100 | 100 | 100 | - |

The preparation process is as follows:
(1) Preparation of an API solution: A prescribed amount of active pharmaceutical ingredient was weighed and diethylene glycol monoethyl ether (or dimethyl sulfoxide) and PEG 400 were added. The mixture was stirred at 50°C for 10 minutes to dissolve the active pharmaceutical ingredient.
(2) Preparation of a carbomer solution: A prescribed amount of carbomer was weighed, which was allowed to fully swell using purified water.
(3) Preparation of gel: The API solution was added to the prepared carbomer solution with stirring (stirring speed 205 rpm). After complete addition, the mixture was stirred for additional 3 minutes, then brought to its final volume with the remaining quantity of water and stirred for 5 minutes (stirring speed 900 rpm).

### Example 12: Organogel

The organogel formulation is shown in Table 12 below.

**Table 12**

| **No.** | **12A** | **12B** | **12C** | **Role** |
|---|---|---|---|---|
| Active pharmaceutical ingredient / % | 1.5 | 1.5 | 1.5 | API |
| Diethylene glycol monoethyl ether / % | 20.0 | 20.0 | 20.0 | Permeation enhancer, vehicle |
| Polyethylene glycol 400 / % | 77.5 | 40.0 | / | Permeation enhancer, vehicle |
| Propylene glycol / % | / | 37.5 | 77.5 | Moisturizing agent, vehicle |
| Carbomer / % | 1.0 | 1.0 | 1.0 | Gel matrix |
| Total / % | 100.0 | 100.0 | 100.0 | - |

The preparation process is as follows:
(1) Preparation of an API solution: Prescribed amounts of active pharmaceutical ingredient and diethylene glycol monoethyl ether (Transcutol P) were weighed and stirred at 50°C for 20 minutes.
(2) Preparation of a carbomer Solution: A prescribed amount of carbomer was weighed, which was allowed to fully swell using prescribed amounts of polyethylene glycol 400 or propylene glycol.
(3) Preparation of gel: The API solution was added to the prepared carbomer solution with stirring (stirring speed 205 rpm). After complete addition, the mixture was stirred for additional 3 minutes (stirring speed 900 rpm).

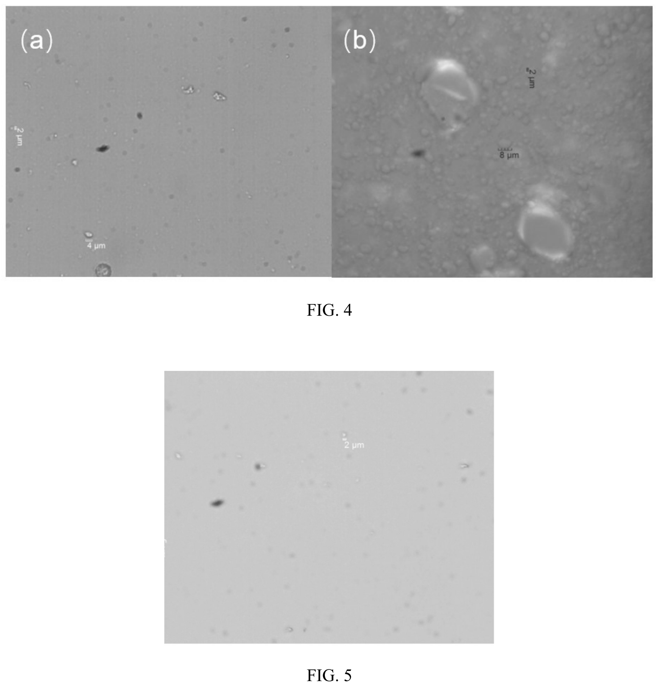

Sample 12A (under initial conditions and accelerated conditions at 40°C and 75% RH for 6 months) was observed under a microscope according to the method of Example 3. The results, as shown in parts (a) and (b) of FIG. 4, indicate no crystallization in the organogel.

### Example 13: Liniment

The liniment formulation is shown in Table 13 below.

**Table 13**

| **No.** | **13A** | **13B** | **13C** | **Role** |
|---|---|---|---|---|
| Active pharmaceutical | 2.0 | 2.0 | 2.0 | API |
| ingredient / % | | | | |
| Diethylene glycol monoethyl ether / % | 20.0 | 20.0 | 20.0 | Permeation enhancer, vehicle |
| Propylene glycol / % | / | 30.0 | / | Permeation enhancer, vehicle |
| Ethanol / % | / | / | 60.0 | Permeation enhancer, vehicle |
| Polyethylene glycol 400 / % | 78.0 | 48.0 | 18.0 | Permeation enhancer, vehicle |
| Total / % | 100.0 | 100.0 | 100.0 | - |

The preparation process is as follows:
Prescribed amounts of active pharmaceutical ingredient and permeation enhancer were weighed. A prescribed amount of permeation enhancer was mixed uniformly, then the active pharmaceutical ingredient was added. The mixture was stirred at 50±5°C until completely dissolved. In this example, the liniment is in a solution state.

Sample 13A was observed under a microscope under initial conditions according to the method described in Example 3. The results, as shown in FIG. 5, indicate no crystals in the field of view.

### Comparative Example 1

In comparison with Example 1, the difference lies in its formulation, as shown in Table 14:

**Table 14**

| **No.** | **1a** | **Role** |
|---|---|---|
| Active pharmaceutical ingredient / % | 0.6 | API |
| Diethylene glycol monoethyl ether / % | 10.0 | Permeation enhancer, vehicle |
| Polyethylene glycol 400 / % | 39.4 | Permeation enhancer, vehicle |
| Polyethylene glycol 3350 / % | 50.0 | Ointment matrix |
| Total / % | 100.0 | - |

### Comparative Example 2

Compared with Sample 1A of Example 1, the difference lies in replacing the active pharmaceutical ingredient with Tofacitinib citrate (Batch No.: HY-170-35-20200327; Supplier: Shanghai Haoyuan Chemexpress Co.,Ltd.), and the resulting sample is designated as 2a.

### Comparative Example 3

The active pharmaceutical ingredient was formulated in a mixture of 50% PEG400, 10% diethylene glycol monoethyl ether, and 40% PEG3350 (50% aqueous solution), and the resulting sample was designated as 3a.

### Effect Example 1: Microscopic observation results

Microscopic results indicate that from part (a) of FIG. 1, part (a) of FIG. 4, and FIG. 5, the ointment, organogel, and liniment show no crystallization under initial conditions. From part (b) of FIG. 1, it can be observed that the ointment remains stable for an extended period without crystallization, and the product exhibits uniform texture with good skin feel. From part (b) of FIG. 4, it can be observed that no significant large crystal particles precipitated when the organogel was stored to test for stability, with a particle size not exceeding 180 µm. From FIGs. 2 and 3, it can be observed that both the cream and hydrogel show no significant precipitation of large crystals under initial conditions, with particle sizes smaller than 180 µm.

### Effect Example 2: In vitro release data of ointment

The release amount was calculated using the external standard method, and the test method for *in vitro* release is shown in Table 15 below:

**Table 15**

| **Project** | **Proposed *in vitro* release method** |
|---|---|
| *In vitro* release method | Franz diffusion cell; 600 rpm |
| Receptor fluid | pH 7.4 phosphate buffer + 1.0% SDS-ethanol (90:10) |
| Sample loading/Temperature | 0.3g/32±1°C |
| Artificial membrane | 0.22 µm organic nylon 66 filter membrane |
| *In vitro* release volume | 12mL |
| *In vitro* release time | 15min, 30min, 60min, 120min, 180min |
| Sample/Replenishment volume | 12mL |
| Detection method | HPLC method |
| | Mobile phase: 0.02 mol/L sodium phosphate dibasic dodecahydrate buffer-acetonitrile (60:40) |
| | Chromatographic column: ZORBAX SB-C18 (150mm*4.6mm, 5 µm) |
| | Flow rate: 1.0 mL/min; Column temperature: 30°C; Detection wavelength: 255 nm; Injection volume: 10 µL. |
| Limit | 75%-133.33% |

Samples 3A, 3B, 3C, and 3D were subjected to the *in vitro* release testing method described above to investigate the *in vitro* release of ointments of different specifications. The results are presented in Table 16 below:

**Table 16**

| **No.** | **Time (h)** | **0.25** | **0.5** | **1** | **2** | **3** |
|---|---|---|---|---|---|---|
| | **Square root of time (h)** | **0.50** | **0.71** | **1.00** | **1.41** | **1.73** |
| 3A | **Drug release amount per unit area (µg/cm²)** | 1344.40 | 2730.53 | 5986.69 | 13129.47 | 13826.27 |
| 3B | **Drug release amount per unit area (µg/cm²)** | 3991.47 | 7994.80 | 17231.73 | 37733.47 | 39873.04 |
| 3C | **Drug release amount per unit area (µg/cm²)** | 11609.32 | 23326.58 | 50551.70 | 110363.32 | 121934.47 |
| 3D | **Drug release amount per unit area (µg/cm²)** | 17270.64 | 34528.02 | 72631.58 | 142585.46 | 170908.87 |

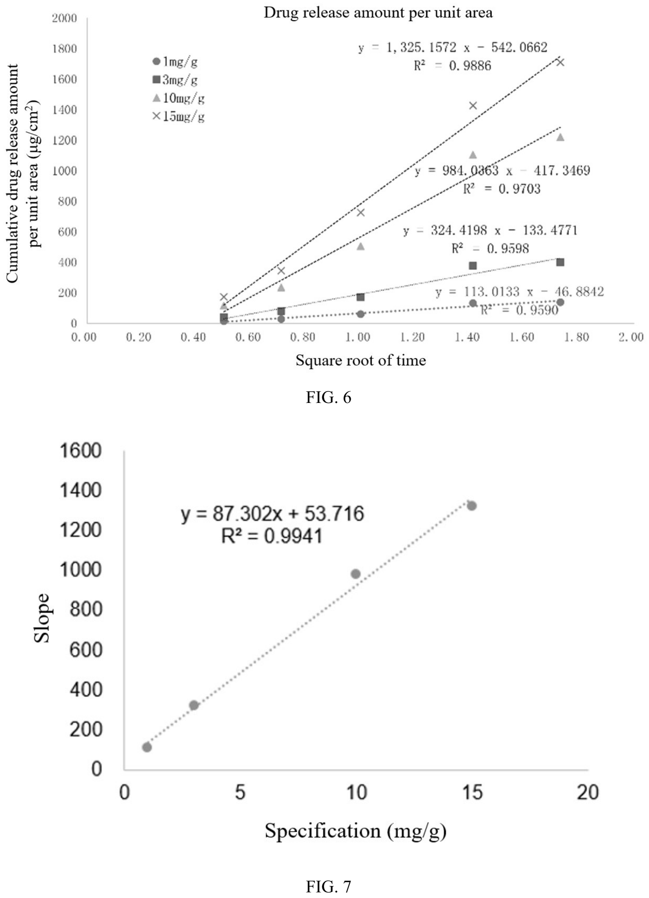

Based on Table 16, a trend line was plotted with the square root of time as the abscissa and the drug release amount per unit area (cumulative) as the ordinate, as shown in FIG. 6; the slope of the trend line represents the release rate, as indicated in Table 17 below:

**Table 17**

| **No.** | **Specification (mg/g)** | **Release rate (slope)** |
|---|---|---|
| 3A | 1 (0.1%) | 113.0133 |
| 3B | 3 (0.3%) | 324.4198 |
| 3C | 10 (1.0%) | 984.0363 |
| 3D | 15 (1.5%) | 1325.1572 |

A curve was plotted with the specification as the abscissa and the release rate (slope) as the ordinate, as shown in FIG. 7. Linear regression analysis yielded a coefficient of determination (R²) greater than 0.90, indicating that the release rates for different specifications exhibit a linear relationship.

The results indicate that as the content of the active pharmaceutical ingredient increases, both the release amount and release rate of the ointment increase.

### Effect Example 3: In vitro percutaneous permeation of ointment

An *in vitro* percutaneous permeation test of ointments was conducted on samples 1C, 1D, 3A, 3B, 3C, 3D, and 1a.

The testing method is as follows:
Before use, the skin was treated with normal saline and evenly cut and allocated to the preparations of each preparation (6 samples per specification). A defatted cotton swab was used to apply the ointment containing the active pharmaceutical ingredient onto the stratum corneum side of the skin. Based on the diameter of the diffusion cell, the applied dose was approximately 0.2g.

A Franz diffusion cell with a 2 cm diameter was used, with 10% ethanol in normal saline as the receptor fluid, and the receptor chamber volume was approximately 7 mL. The prepared pig skin was fixed onto the Franz diffusion cell, with the stratum corneum facing upward toward the donor chamber and the dermis facing downward toward the receptor chamber. The ointment was uniformly applied onto the stratum corneum surface of the skin at an amount of 0.2 g, and the receptor chamber was filled with degassed receptor fluid. The diffusion cell was positioned within a constant temperature water bath maintained at (32±1)°C. A magnetic stir bar was used to continuously stir the receptor fluid at a rotation speed at 200 rpm. The entire receptor fluid was removed at time points of 2 hours, 4 hours, 6 hours, 8 hours, 10 hours, and 24 hours, and an equivalent volume of isothermal receptor fluid was replenished. The test receptor fluid was stored at 5°C. The experiment was terminated at 24 hours, and the skin sections on each cell were subjected to processing.

Receptor fluid processing: Upon completion of the *in vitro* percutaneous permeation experiment, precisely 200 µL of the receiver fluid was pipetted into a 96-well plate. To this, 200 µL of an internal standard solution (50% methanol aqueous solution of diclofenac sodium, 92.76 ng/mL) was added. The mixture was then vortexed for 10 minutes to ensure thorough mixing. The mixture was centrifuged (5°C, 4000 g) for 15 minutes. Precisely 200µL of the resulting supernatant was accurately pipetted into a separate 96-well plate to obtain the test sample.

Skin processing (residual ointment on skin surface): Upon completion of the *in vitro* percutaneous permeation experiment, the transdermal diffusion cell was disassembled. The skin surface was visually inspected for any apparent residual ointment. A dropper was then used to gently aspirate any liquid present on the skin surface. The residual ointment was aspirated as completely as possible and transferred to a volumetric flask. Subsequently, 2 mL of methanol was transferred to the donor chamber using a 1 mL pipette. The skin surface was gently scraped with a small spatula to maximize the dissolution of the residual drug from the skin surface into methanol. The resulting wash solution was then transferred to the aforementioned volumetric flask. The above procedure was repeated six times. The contents were then subjected to ultrasonication for 15 minutes, followed by cooling, and finally diluted to the final volume with methanol. The collected ointment wash solution was stored at 5°C. Precisely 10 µL of the collected ointment wash solution (analysis sample) was accurately aspirated and diluted by adding 990 µL of blank matrix. The resulting mixture was vortexed for 3 minutes to ensure thorough mixing. Subsequently, 200 µL of the above analysis sample was aspirated into a 1.5 mL EP tube. To this, 600 µL of the internal standard solution (50% methanol aqueous solution of diclofenac sodium, 92.76 ng/mL) was added. The mixture was then vortexed for 10 minutes to ensure thorough mixing. The mixture was centrifuged (5°C, 12000 rpm) for 5 minutes. Precisely 200µL of the resulting supernatant was accurately pipetted into a 96-well plate to obtain the test sample.

Skin processing (skin extraction solution): Following the processing of the residual ointment on the skin surface, the skin was removed from the cell. Residual ointment on the skin surface was washed off, and any residual vehicle on the skin surface was blotted dry using filter paper. The peripheral skin portion that did not directly contact the ointment was excised using scissors, retaining only the skin section corresponding to the orifice area of the cell (i.e., approximately 3.14cm²). The processed skin section was weighed, placed into an EP tube, and sealed for storage. The skin section was fragmented and frozen in liquid nitrogen for at least 1 hour. Subsequently, the frozen skin was transferred to a freeze mill and ground into a powder. The grinding ball was then directly transferred into a 50 mL EP tube using a total of 25 mL of methanol dispensed in multiple small volumes. The contents were then subjected to ultrasonication, followed by shaking to homogenize the mixture. 1 mL of the resulting mixture was aspirated and transferred into a 1.5 mL EP tube, yielding the skin extraction solution, which was sealed and stored at 5°C. Precisely 200 µL of the skin extraction solution was aspirated into a 1.5 mL EP tube. To this, 200 µL of the internal standard solution (50% methanol aqueous solution of diclofenac sodium, 92.76 ng/mL) was added. The mixture was then vortexed for 10 minutes to ensure thorough mixing. The mixture was centrifuged (5°C, 12000 rpm) for 5 minutes. Precisely 200µL of the resulting supernatant was accurately pipetted into a 96-well plate to obtain the test sample.

The concentration of the active pharmaceutical ingredient in the receptor fluid, the residual ointment on the skin surface, and the skin extraction solution was determined using the LC-MS/MS method. The cumulative permeation amount and the skin retention amount were calculated.

The results of the transdermal experiment are presented in Table 18 below.

**Table 18**

| **No.** | **Skin retention amount per unit area (µg/cm²)** | **Percentage of drug retained relative to initial loading dose (%)** | **24-Hour permeation amount (µg /cm²)** | **Percentage of drug permeated relative to initial loading dose (%)** | **Retention/permeation ratio** |
|---|---|---|---|---|---|
| 1a | 0.5971 | 0.0956 | 0.039550 | 0.0114 | 15.10 |
| 1C | 0.8046 | 0.0647 | 0.045960 | 0.0066 | 17.51 |
| 1D | 6.373 | 0.329 | 0.106500 | 0.00979 | 59.84 |
| 3A | 0.02713 | 0.0158 | 0.018160 | 0.000292 | 47.93 |
| 3B | 0.08375 | 0.0165 | 0.037690 | 0.000461 | 36.02 |
| 3C | 0.1511 | 0.00893 | 0.000566 | 0.000491 | 18.18 |
| 3D | 0.6899 | 0.0267 | 0.002325 | 0.000966 | 27.54 |

Result analysis: The *in vitro* transdermal test results for samples 1a, 1C, and 1D indicate that the skin retention amount per unit area increases as the mass ratio of polyethylene glycol 400 to diethylene glycol monoethyl ether and the mass ratio of polyethylene glycol 3350 to diethylene glycol monoethyl ether are reduced. A comparison of the results for samples 3A, 3B, 3C, and 3D indicates that the skin retention amount of the ointment exhibits a certain correlation with the drug loading, wherein the skin retention amount per unit area increases as the drug loading increases. The composition of the present disclosure demonstrate a significantly greater retention to permeation ratio, indicating a lower amount entering the bloodstream and, thus, a reduced systemic risk.

### Effect Example 4: Results for stability study

Stability studies were conducted on samples 3B, 3C, and 3D.

The analytical method is as follows:
(1) Shape: The physical appearance of the ointment was visually inspected.
(2) pH value: The ointment was diluted 10-fold with purified water, which was freshly boiled and cooled to room temperature. After shaking to disperse the sample, the pH value was determined using a pH meter.
(3) Related substances (impurities, species are shown in Table 19 below): The related substances were determined in accordance with the high-performance liquid chromatography method (General Guideline 0512, Part IV, Chinese Pharmacopoeia 2020 Edition). The related substance is determined as follows: The chromatographic column is a Waters Xselect Hss T3 C18 column (4.6*150mm, 3.5µm). The mobile phase A is an aqueous solution of 10mM ammonium acetate (prepared by precisely transferring 0.77g of ammonium acetate into 1000mL of purified water, mixing, and degassing) and the mobile phase B is acetonitrile. Gradient elution is performed according to Table 20, which employs a flow rate of 1.0 mL/min, a column temperature of 15°C, a detection wavelength of 254 nm, and a run time of 45 minutes. The rinse solution is 50% acetonitrile in water.

**Table 19**

| **Impurity** | **Chemical formula** | **Molecular weight** |
|---|---|---|
| Known impurity 1 | C₂₄H₂₉N₅O₃ | 435.52 |
| Known impurity 2 | C₃₆H₄₈N₈O₃ | 640.82 |
| Known impurity 3 | C₂₉H₄₄N₈O₂ | 536.71 |
| Known impurity 4 | C₁₄H₁₁ClN₄O | 286.72 |
| Known impurity 5 | C₂₆H₃₃N₇O₃ | 491.6 |

**Table 20**

| **Time (min)** | **Mobile phase A (%)** | **Mobile phase B (%)** |
|---|---|---|
| 0 | 90 | 10 |
| 5 | 70 | 30 |
| 25 | 60 | 40 |
| 35 | 10 | 90 |
| 40 | 10 | 90 |
| 40.1 | 90 | 10 |
| 45 | 90 | 10 |

(3) Content: The content was determined by high-performance liquid chromatography (General Guideline 0512, Part IV, Chinese Pharmacopoeia 2020 Edition). The content determination method is as follows: A mobile phase is prepared from 0.02 mol/L di-sodium hydrogen phosphate dodecahydrate buffer solution and acetonitrile in a ratio of 60:40, wherein the buffer solution is prepared by weighing 7.20 g of di-sodium hydrogen phosphate dodecahydrate, dissolving it in 1000 mL of water, adjusting the pH to 7.80±0.05 using phosphoric acid, and filtering the resulting solution under vacuum using a 0.45 µm aqueous filter membrane. The separation is conducted via isocratic elution with a column temperature maintained at 30°C, a detection wavelength of 255 nm, a flow rate of 1.0 mL per minute, and an injection volume of 10 µL. The method for content determination is also applicable to the determination of butylated hydroxytoluene content.
(4) Viscosity: Viscosity is measured using a Brookfield viscometer.
(5) Microscopic observation: A clean glass slide was taken and an appropriate amount of the ointment (approximately 0.01g per sampling) was applied onto the slide, which was covered with a coverslip. The coverslip was gently pressed. The ointment was observed under a microscope with an eyepiece magnification of 10X, an objective magnification of 40X, and polarized light at 300°.

Result is as follows:
A. The results of the 30-day stability study under high temperature at 50°C and illumination of ≥1.2×10⁶ lux·hr are shown in Table 21.

**Table 21**

| **No.** | **Test item** | **0 days** | **High temperature: 50°C** | | | **Illumination: ≥1.2×10⁶ lux·hr 30 days** |
|---|---|---|---|---|---|---|
| | | | **5 days** | **10 days** | **30 days** | |
| 3B | Appearance | Pale yellow ointment | Pale yellow ointment | Pale yellow ointment | Pale yellow ointment | Pale yellow ointment |
| | pH value | 6.1 | 5.4 | 5.6 | 5.9 | 6.3 |
| | Known impurity 1 / % | ND | ND | ND | ND | ND |
| | Known impurity 2/% | ND | ND | ND | ND | ND |
| | Known impurity 3 / % | ND | ND | ND | ND | ND |
| | Known impurity 4 / % | 0.11 | 0.11 | 0.10 | 0.10 | 0.12 |
| | Known impurity 5 / % | ND | 0.07 | 0.10 | 0.17 | ND |
| | Other maximum single impurity | 0.05 | 0.16 | 0.17 | 0.12 | 0.17 |
| | Total impurities | 0.16 | 0.39 | 0.44 | 0.67 | 0.25 |
| | Content / % | 100.5 | 100.4 | 100.2 | 100.7 | 100.3 |
| | Viscosity / Pa·s | 50733 | 59733 | 41333 | 66900 | 54000 |
| | Microscopic observation | No obvious crystal particles | No obvious crystal particles | No obvious crystal particles | No obvious crystal particles | No obvious crystal particles |
| 3C | Appearance | Pale yellow ointment | Pale yellow ointment | Pale yellow ointment | Pale yellow ointment | Pale yellow ointment |
| | pH value | 6.0 | 5.4 | 5.8 | 5.8 | 6.3 |
| | Known impurity 1 / % | ND | ND | ND | ND | ND |
| | Known impurity 2/% | ND | ND | ND | ND | ND |
| | Known impurity 3 / % | ND | ND | ND | ND | ND |
| | Known impurity 4 / % | 0.11 | 0.11 | 0.10 | 0.10 | 0.11 |
| | Known impurity 5/% | ND | 0.06 | 0.08 | 0.14 | 0.05 |
| | Other maximum single impurity | ND | 0.05 | 0.08 | 0.08 | ND |
| | Total impurities | 0.11 | 0.21 | 0.26 | 0.44 | 0.15 |
| | Content / % | 102.2 | 99.2 | 100.9 | 101.7 | 101.2 |
| | Viscosity / Pa·s | 81467 | 54867 | 68267 | 38700 | 78800 |
| | Microscopic observation | No obvious crystal particles | No obvious crystal particles | No obvious crystal particles | No obvious crystal particles | No obvious crystal particles |
| 3D | Appearance | Pale yellow ointment | Pale yellow ointment | Pale yellow ointment | Pale yellow ointment | Pale yellow ointment |
| | pH value | 5.7 | 5.5 | 5.8 | 5.7 | 6.3 |
| | Known impurity 1 / % | ND | ND | ND | ND | ND |
| | Known impurity 2/% | ND | ND | ND | ND | ND |
| | Known impurity 3 / % | ND | ND | ND | ND | ND |
| | Known impurity 4 / % | 0.11 | 0.10 | 0.10 | 0.09 | 0.10 |
| | Known impurity 5 / % | ND | 0.06 | 0.08 | 0.11 | ND |
| | Other maximum single impurity | ND | 0.05 | 0.07 | 0.07 | ND |
| | Total impurities | 0.11 | 0.21 | 0.25 | 0.33 | 0.10 |
| | Content | 98.7% | 100.4% | 101.2% | 101.9% | 100.2% |
| | Viscosity / Pa·s | 59267 | 60067 | 86867 | 59267 | 67667 |
| | Microscopic | No obvious | No | No | No | No obvious |
| | observation | crystal particles | obvious crystal particles | obvious crystal particles | obvious crystal particles | crystal particles |

| | | | | | | |
|---|---|---|---|---|---|---|
| Note: "ND" indicates below the reporting limit of 0.05% or not detected, "-" indicates not applicable. | | | | | | |

B. The results of the 3-month stability study under accelerated conditions (40°C, 75% RH) are shown in Table 22.

**Table 22**

| **No.** | **Test item** | | **0 month** | **1 month** | **3 months** |
|---|---|---|---|---|---|
| | Appearance | | The product is a pale yellow ointment. | The product is a pale yellow ointment. | The product is a pale yellow ointment. |
| | Related substance | Known impurity 1 / % | <0.02% | <0.02% | <0.02% |
| | | Known impurity 2 / % | <0.02% | <0.02% | <0.02% |
| 3B | | Known impurity 3 / % | <0.02% | <0.02% | <0.02% |
| | | Known impurity 4 / % | 0.09% | 0.09% | 0.10% |
| | | Known impurity 5 / % | <0.02% | 0.10% | 0.19% |
| | | Other individual impurities | <0.05% | 0.05% | 0.16% |
| | | Total impurities | 0.09% | 0.24% | 0.59% |
| | pH | | 5.9 | 6.5 | 5.7 |
| | Butylated hydroxytoluene | | 0.10% | 0.10% | 0.10% |
| | Content | | 98.1% | 99.1% | 98.7% |
| | Appearance | | The product is a pale yellow ointment. | The product is a pale yellow ointment. | The product is a pale yellow ointment. |
| | Related substance | Known impurity 1 / % | <0.02% | <0.02% | <0.02% |
| | | Known impurity 2 / % | <0.02% | <0.02% | <0.02% |
| | | Known impurity 3 /% | <0.02% | <0.02% | <0.02% |
| 3C | | Known impurity 4 / % | 0.09% | 0.09% | 0.10% |
| | | Known impurity 5 / % | <0.02% | 0.09% | 0.15% |
| | | Other individual impurities | <0.05% | <0.05% | 0.15% |
| | | Total impurities | 0.09% | 0.18% | 0.53% |
| | pH | | 6.1 | 6.4 | 5.7 |
| | Butylated hydroxytoluene | | 0.10% | 0.10% | 0.10% |
| | Content | | 99.2% | 100.6% | 100.4% |
| 3D | Appearance | | The product is a pale yellow ointment. | The product is a pale yellow ointment. | The product is a pale yellow ointment. |
| | Related substance | Known impurity 1 / % | <0.02% | <0.02% | <0.02% |
| | | Known impurity 2 / % | <0.02% | <0.02% | <0.02% |
| | | Known impurity 3 /% | <0.02% | <0.02% | <0.02% |
| | | Known impurity 4 / % | 0.09% | 0.09% | 0.10% |
| | | Known impurity 5 / % | <0.02% | 0.09% | 0.18% |
| | | Other individual impurities | <0.05% | <0.05% | 0.11% |
| | | Total impurities | 0.09% | 0.18% | 0.50% |
| | pH | | 6.0 | 6.6 | 5.6 |
| | Butylated hydroxytoluene | | 0.10% | 0.10% | 0.10% |
| | Content | | 98.5% | 99.5% | 99.4% |

C. The results of the 3-month stability study under long-term conditions (25°C, 60% RH) are shown in Table 23.

**Table 23**

| **No.** | **Test item** | | **0 month** | **1 month** | **3 months** |
|---|---|---|---|---|---|
| | Appearance | | The product is a pale yellow ointment. | The product is a pale yellow ointment. | The product is a pale yellow ointment. |
| | Related substance | Known impurity 1 / % | <0.02% | <0.02% | <0.02% |
| | | Known impurity 2/% | <0.02% | <0.02% | <0.02% |
| 3B | | Known impurity 3/% | <0.02% | <0.02% | <0.02% |
| | | Known impurity 4 / % | 0.09% | 0.09% | 0.10% |
| | | Known impurity 5/% | <0.02% | <0.05% | 0.06% |
| | | Other individual impurities | <0.05% | <0.05% | <0.05% |
| | | Total impurities | 0.09% | 0.09% | 0.16% |
| | pH | | 5.9 | 6.6 | 5.7 |
| | Butylated hydroxytoluene | | 0.10% | 0.10% | 0.10% |
| | Content | | 98.1% | 98.9% | 99.2% |
| | Appearance | | The product is a pale yellow ointment. | The product is a pale yellow ointment. | The product is a pale yellow ointment. |
| | Related substance | Known impurity 1 / % | <0.02% | <0.02% | <0.02% |
| | | Known impurity 2/% | <0.02% | <0.02% | <0.02% |
| | | Known impurity 3/% | <0.02% | <0.02% | <0.02% |
| 3C | | Known impurity 4/% | 0.09% | 0.09% | 0.10% |
| | | Known impurity 5 / % | <0.02% | <0.05% | 0.06% |
| | | Other individual impurities | <0.05% | <0.05% | <0.05% |
| | | Total impurities | 0.09% | 0.09% | 0.16% |
| | pH | | 6.1 | 6.6 | 5.8 |
| | Butylated hydroxytoluene | | 0.10% | 0.10% | 0.10% |
| | Content | | 99.2% | 99.2% | 99.6% |
| 3D | Appearance | | The product is a pale yellow ointment. | The product is a pale yellow ointment. | The product is a pale yellow ointment. |
| | Related substance | Known impurity 1 / % | <0.02% | <0.02% | <0.02% |
| | | Known impurity 2/% | <0.02% | <0.02% | <0.02% |
| | | Known impurity 3/% | <0.02% | <0.02% | <0.02% |
| | | Known impurity 4/% | 0.09% | 0.09% | 0.10% |
| | | Known impurity 5/% | <0.02% | <0.05% | 0.07% |
| | | Other individual impurities | <0.05% | <0.05% | <0.05% |
| | | Total impurities | 0.09% | 0.09% | 0.17% |
| | pH | | 6.0 | 6.4 | 5.6 |
| | Butylated hydroxytoluene | | 0.10% | 0.10% | 0.10% |
| | Content | | 98.5% | 99.0% | 99.6% |

D. The results of the 2-month stability study during the use period (32°C, 75% RH) are shown in Table 24.

**Table 24**

| **No.** | **Test item** | | **0 days** | **15 days** | **30 days** | **60 days** |
|---|---|---|---|---|---|---|
| | Appearance | | The product is a pale yellow ointment. | The product is a pale yellow ointment. | The product is a pale yellow ointment. | The product is a pale yellow ointment. |
| | Related substance | Known impurity 1 / % | ND | ND | ND | ND |
| | | Known impurity 2 / % | ND | ND | ND | ND |
| 3B | | Known impurity 3 / % | ND | ND | ND | ND |
| | | Known impurity 4 / % | 0.11 | 0.10 | 0.10 | 0.10 |
| | | Known impurity 5 / % | ND | ND | 0.06 | 0.07 |
| | | Other individual impurities | 0.05 | 0.11 | 0.13 | 0.14 |
| | | Total impurities | 0.16 | 0.26 | 0.34 | 0.31 |
| | pH | | 6.1 | 5.8 | 6.1 | 6.1 |
| | Content / % | | 100.5 | 101.7 | 100.8 | 101.6 |
| | Viscosity / Pa·s | | 50733 | 59667 | 67200 | 69400 |
| | Appearance | | The product is a pale yellow ointment. | The product is a pale yellow ointment. | The product is a pale yellow ointment. | The product is a pale yellow ointment. |
| | Related substance | Known impurity 1 / % | ND | ND | ND | ND |
| | | Known impurity 2 / % | ND | ND | ND | ND |
| | | Known impurity 3 / % | ND | ND | ND | ND |
| 3C | | Known impurity 4 / % | 0.11 | 0.10 | 0.10 | 0.10 |
| | | Known impurity 5 / % | ND | ND | 0.06 | 0.07 |
| | | Other individual impurities | 0.05 | 0.11 | 0.13 | 0.14 |
| | | Total impurities | 0.16 | 0.26 | 0.34 | 0.31 |
| | pH | | 6.1 | 5.8 | 6.1 | 6.1 |
| | Content (%) | | 100.5 | 101.7 | 100.8 | 101.6 |
| | Viscosity (Pa·s) | | 50733 | 59667 | 67200 | 69400 |
| 3D | Appearance | | The product is a pale yellow ointment. | The product is a pale yellow ointment. | The product is a pale yellow ointment. | The product is a pale yellow ointment. |
| | Related substance | Known impurity 1 / % | ND | ND | ND | ND |
| | | Known impurity 2 / % | ND | ND | ND | ND |
| | | Known impurity 3 / % | ND | ND | ND | ND |
| | | Known impurity 4 / % | 0.11 | 0.10 | 0.10 | 0.10 |
| | | Known impurity 5 / % | ND | ND | 0.06 | 0.07 |
| | | Other individual impurities | 0.05 | 0.11 | 0.13 | 0.14 |
| | | Total impurities | 0.16 | 0.26 | 0.34 | 0.31 |
| | pH | | 6.1 | 5.8 | 6.1 | 6.1 |
| | Content (%) | | 100.5 | 101.7 | 100.8 | 101.6 |
| | Viscosity (Pa·s) | | 50733 | 59667 | 67200 | 69400 |

The results from Tables 21-24 indicate that, compared to the 0-day results, the drug compositions of different specifications showed no significant changes in properties such as appearance, pH, content, related substances, and viscosity after 30 days under influencing factors, as well as 3 months under accelerated and long-term conditions. Furthermore, the stability during use was evaluated, and the results were favorable. In summary, the pharmaceutical composition of the present disclosure exhibits excellent stability when used for preparations.

### Effect Example 5: Pharmacokinetic (PK) study results

### Experimental method:

Twenty-four (12 per sex) male and female Bama miniature pigs were divided into 5 groups. The ointment was applied to an estimated 10% of the animal's body surface area.

Animals in group 1 and group 2 received a single topical application of sample 2A and sample 2B, respectively. Plasma, stratum corneum, epidermis, and dermis samples were collected before administration (0 hour, plasma only) and at 0.5, 1, 4, 8, 12, and 24 hours after administration.

Group 3 animals were topically administered ointment sample 2B once daily for 7 consecutive days. Plasma samples were collected on day 1 and day 7 before administration (0) and 0.5, 1, 4, 8, 12, and 24 hours after administration; and on days 3, 4, 5, and 6 before administration (0). Stratum corneum, epidermis, and dermis samples were collected on Day 7 at 0.5, 1, 4, 8, 12, and 24 hours after administration.

Group 4 animals received a single topical application of ointment sample 2a. Plasma, stratum corneum, epidermis, dermis, and subcutaneous tissue samples were collected before administration (0 hour, plasma only) and at 0.5, 1, 4, 8, 12, and 24 hours after administration.

Group 5 animals received a single topical application of sample 3a (administered at 25 µL/m² with a concentration of 6 mg/mL), and blood and skin samples were collected before administration (0) and at 0.5, 1, 3, and 6 hours after administration. Blood is obtained through venipuncture and collected in a tube containing sodium heparin as an anticoagulant. The exposed skin was washed with soap and water, then the skin sample was collected. At each time point, punch biopsy tissue samples extending from the epidermal layer to the muscle layer were procured from the skin at the site of administration. The epidermis, dermis, subcutaneous tissue, and subcutaneous muscle were rapidly separated, weighed, and frozen in dry ice. Subsequently, the epidermis and the dermis were homogenized in normal saline at a ratio of 1:3 (w/v). The homogenized samples were extracted using three volumes of acetonitrile and quantified against a standard curve utilizing an LC-MS/MS system.

The concentrations of the active pharmaceutical ingredient in plasma, stratum corneum, epidermis, and dermis samples were determined using a high-performance liquid chromatography-ultraviolet detection (LC-MS/MS) method.

**Table 25**

| No.: 2A (group 1) | | | | | | | |
|---|---|---|---|---|---|---|---|
| Dosage: 4 µg/cm², day 1 | | | | | | | |
| Location | | Stratum corneum | Epidermi s | Dermis | | Plasma | |
| Cmax (ng/mL or ng/g) | | 0.622 | 1040 | 137 | | ND | |
| Tmax (h) | | 4.42 | 3.17 | 4.42 | | ND | |
| T₄ (h) | | ND | ND | ND | | ND | |
| AUC0-last (h*ng/mL or ng*h/g) | | 5.28 | 8610 | | 638 | ND | |
| AUC0-inf (h*ng/mL or ng*h/g) | | ND | ND | | ND | ND | |
| No.: 2B (Group 2) | | | | | | | |
| Dosage: 12 µg/cm², day 1 | | | | | | | |

| Location | | Stratum corneum | Epidermi s | | Dermis | Plasma | |
|---|---|---|---|---|---|---|---|
| Cmax (ng/mL or ng/g) | | 1.31 | 2000 | | 176 | ND | |
| Tmax (h) | | 6.92 | 4.92 | | 5.58 | ND | |
| T1/2 (h) | | ND | ND | | ND | ND | |
| AUC0-last (h*ng/mL or ng*h/g) | | 14.4 | 20500 | | 2210 | ND | |
| AUC0-inf (h*ng/mL or ng*h/g) | | ND | ND | | ND | ND | |

| No.: 2B (Group 3) | | | | | | | |
|---|---|---|---|---|---|---|---|
| Dosage: 12 µg/cm², day 1 | | | | | | | |
| Location | | Stratum corneum | Epidermi s | | Dermis | Plasma | |
| Cₘₐₓ (ng/mL or ng/g) | | -- | -- | | -- | ND | |
| Tₘₐₓ (h) | | -- | -- | | -- | ND | |
| T_{1/2} (h) | | -- | -- | | -- | ND | |
| AUC0-last (h*ng/mL or ng*h/g) | | -- | -- | | -- | ND | |
| AUC0-inf (h*ng/mL or ng*h/g) | | -- | -- | | -- | ND | |

| No.: 2B (Group 3) | | | | | | | |
|---|---|---|---|---|---|---|---|
| Dosage: 12 µg/cm², day 7 | | | | | | | |
| Location | | Stratum corneum | Epidermi s | | Dermis | Plasma | |
| Cₘₐₓ (ng/mL or ng/g) | | 1.81 | 1890 | | 202 | 0.435 | |
| Tₘₐₓ (h) | | 4.67 | 14.8 | | 3.08 | 8.00 | |
| T_{1/2} (h) | | ND | ND | | ND | ND | |
| AUC0-last (h*ng/mL or ng*h/g) | | 18.5 | 27800 | | 1570 | 2.99 | |
| AUC0-inf (h*ng/mL or ng*h/g) | | ND | ND | | ND | ND | |

| No.: 2a (group 4) | | | | | | | |
|---|---|---|---|---|---|---|---|
| Dosage: 0.50 mg/cm², day 1 | | | | | | | |
| Location | Stratum corneum | Epidermis | Dermi s | Subcutaneous tissue | | | Plas ma |
| Cₘₐₓ (ng/mL or ng/g) | 37.9 | 491 | 646 | 224 | | | ND |
| Tₘₐₓ (h) | 3.00 | 6.00 | 1.00 | 6.00 | ND | | |
| T_{1/2} (h) | ND | ND | ND | ND | ND | | |
| AUC0-last (h*ng/mL or ng*h/g) | 79.1 | 1661 | 1037 | 679 | ND | | |
| AUC_{0-inf} (h*ng/mL or ng*h/g) | ND | ND | ND | ND | ND | | |

| No.: 3a (group 5) | | | | | | | |
|---|---|---|---|---|---|---|---|
| Dosage: 150 µg/cm² | | | | | | | |
| Location | Epidermis | Dermis | Plasma | | | | |
| AUC0-last (h*ng/mL or ng*h/g) | 107236.9 | 5209.4 | NA | | | | |

| | | | | | | | |
|---|---|---|---|---|---|---|---|
| Note: "h*ng/mL" is the unit for plasma, and "ng*h/g" is the unit for skin tissue. | | | | | | | |

The results of topical skin application showed that comparing 2A and 2B outcomes indicates a positive correlation between AUC in different skin layers and the administered dose. The high AUC0-last of the active pharmaceutical ingredient in the epidermal and dermal layers indicates that it can effectively penetrate the stratum corneum and remain at the therapeutically effective site. Concentrations of the active pharmaceutical ingredient in all plasma samples are below the lower limit of quantification, indicating minimal systemic absorption of the active pharmaceutical ingredient. Furthermore, after 7 consecutive days of once-daily topical application of the ointment at 12 µg/cm², the exposure levels (AUC₀₋ₗₐₛₜ and Cₘₐₓ) in the stratum corneum, epidermis, and dermis of both sexes remained essentially unchanged, indicating no accumulation of the active pharmaceutical ingredient.

Compared with the composition containing tofacitinib (sample 2a) and the composition containing water (sample 3a), the composition of the present disclosure exhibits significantly greater retention of the unit dose of the active pharmaceutical ingredient in both the epidermis and dermis after application. This indicates that the pharmaceutical composition of the present disclosure can more effectively penetrate the stratum corneum to reach the site of action, demonstrating superior permeability.

### Effect Example 6: Results for stability study

A stability study was conducted on sample 3D. The analysis method is as described in Effect Example 4.

The results for the 12-month stability study on sample 3D under long-term conditions (25°C, 60% RH) are shown in Table 26.

**Table 26**

| **No .** | **Test item** | | **6 months** | **9 months** | **12 months** |
|---|---|---|---|---|---|
| | Appearance | | The product is a pale yellow ointment. | The product is a pale yellow ointment. | The product is a pale yellow ointment. |
| | Related substance | Known impurity 1/% | <0.02% | <0.02% | <0.02% |
| | | Known impurity 2/% | <0.02% | <0.02% | <0.02% |
| | | Known impurity 3/% | <0.02% | <0.02% | <0.02% |
| 3D | | Known impurity 4 / % | 0.10% | 0.10% | 0.10% |
| | | Known impurity 5/% | 0.08% | 0.09% | 0.10% |
| | | Other individua l impuritie s | RRT0.73:0.05 % | RRT0.73:0.07 % | RRT0.73:0.05 % |
| | | | | RRT1.12:0.05 % | RRT1.12:0.07 % |
| | | Total impuritie s | 0.23% | 0.31% | 0.37% |
| | pH | | 5.5 | 5.6 | 5.7 |
| | Butylated hydroxytoluene | | 0.10% | 0.10% | 0.10% |
| | Content | | 100.2% | 101.7% | 99.8% |

From the results in Table 26, it can be seen that the pharmaceutical composition of sample 3D obtained in Example 3 shows no significant changes in properties such as appearance, pH, content, related substances, and viscosity after being placed under influencing factors for 6 months, 9 months, and 12 months. Furthermore, the stability during use was evaluated, and the results were favorable. In summary, the pharmaceutical composition of the present disclosure exhibits excellent stability when used for preparations.

While specific embodiments of the present disclosure have been described above, it should be understood by those skilled in the art that these are provided by way of illustration only, and that various changes or modifications may be made to these embodiments without departing from the principle and substance of the present disclosure. Therefore, the scope of the present disclosure is defined by the appended claims.

## Claims

1. A pharmaceutical composition, wherein the pharmaceutical composition comprises an active pharmaceutical ingredient, diethylene glycol monoethyl ether, and polyethylene glycol 400, wherein the pharmaceutical composition does not comprise water;
wherein the mass ratio of diethylene glycol monoethyl ether to polyethylene glycol 400 is 1:(0.5 to 4.0); and the active pharmaceutical ingredient has a structure of formula (I):

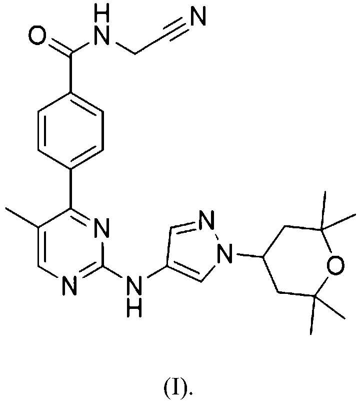

2. The pharmaceutical composition according to claim 1, wherein the active pharmaceutical ingredient is used in an amount of 0.001% to 10%, such as 0.1%, 0.3%, 0.6%, 1.0%, 1.2%, 1.5%, or 2.0%, wherein % refers to the mass percentage of the active pharmaceutical ingredient in the pharmaceutical composition;
and/or, diethylene glycol monoethyl ether is used in an amount of 15% to 40%, such as 20.0%, 23.0%, or 33.8%, wherein % refers to the mass percentage of diethylene glycol monoethyl ether in the pharmaceutical composition;
and/or, polyethylene glycol 400 is used in an amount of 30% to 55%, such as 30.4%, 38.8%, 45.3%, 52.5%, or 52.9%, wherein % refers to the mass percentage of polyethylene glycol 400 in the pharmaceutical composition;
and/or, the mass ratio of the active pharmaceutical ingredient to diethylene glycol monoethyl ether is 1:(10.0 to 250.0), such as 1:13.33, 1:15.33, 1:16.67, 1:16.90, 1:20, 1:23, 1:33.33, 1:66.67, 1:76.67, 1:200, or 1:230;
and/or, the mass ratio of diethylene glycol monoethyl ether to polyethylene glycol 400 is 1:(0.5 to 3.0), such as 1:0.90, 1:1.94, 1:1.97, 1:2.63, or 1:2.65;
and/or, the pharmaceutical composition further comprises polyethylene glycol 3350;
and/or, the pharmaceutical composition further comprises one or more of an antioxidant, preservative, hardening agent, and moisturizing agent.

3. The pharmaceutical composition according to claim 1 or 2, wherein when the pharmaceutical composition comprises polyethylene glycol 3350, then polyethylene glycol 3350 is used in an amount of25.0% to 50%, preferably 25.0% to 45.0%, such as 25.8%, 26.3%, 26.5%, 27%, 27.2%, 30%, 30.5%, 31.2%, 31.4%, 33.8%, or 40%;
and/or, when the pharmaceutical composition comprises polyethylene glycol 3350, then the mass ratio of diethylene glycol monoethyl ether to polyethylene glycol 3350 is 1:(0.5 to 3.6), preferably 1:(0.5 to 3.0), such as 1:1.00, 1:1.29, 1:1.30, 1:1.32, 1:1.33, 1:1.35, 1:1.36, 1:1.37, or 1:2.00;
and/or, the antioxidant is an aromatic amine antioxidant, such as butylated hydroxytoluene;
and/or, the antioxidant is used in an amount of 0.05% to 0.2%, such as 0.1%;
and/or, the preservative is one or more of methylparaben, sorbic acid, and salts thereof, such as methylparaben;
and/or, the preservative is used in an amount of 0.05% to 0.2%, such as 0.1%;
and/or, the hardening agent is one or more of paraffin, beeswax, cetearyl alcohol, white petrolatum, and glyceryl monostearate;
and/or, the hardening agent is used in an amount of 5% to 40%, such as 9.8%, 22.1%, 30.65%, or 37.6%;
and/or, the moisturizing agent is one or more of glycerol, light liquid paraffin, soybean oil, medium-chain triglycerides, sodium hyaluronate, urea, propylene glycol, butylene glycol, panthenol, sodium pyrrolidone carboxylate, and trehalose, preferably propylene glycol;
and/or, the moisturizing agent is used in an amount of 2% to 80%, such as 5.0%, 9.0%, 20.0%, 37.5%, or 77.5%.

4. The pharmaceutical composition according to any one of claims 1 to 3, wherein the pharmaceutical composition comprises the active pharmaceutical ingredient, diethylene glycol monoethyl ether, polyethylene glycol 400, and polyethylene glycol 3350, wherein the pharmaceutical composition does not comprise water;
wherein the mass ratio of diethylene glycol monoethyl ether to polyethylene glycol 400 is 1:(0.5 to 4.0), and the mass ratio of diethylene glycol monoethyl ether to polyethylene glycol 3350 is 1:(0.5 to 3.6);
preferably, the pharmaceutical composition comprises the active pharmaceutical ingredient, diethylene glycol monoethyl ether, polyethylene glycol 400, and polyethylene glycol 3350, wherein the pharmaceutical composition does not comprise water;
wherein the mass ratio of diethylene glycol monoethyl ether to polyethylene glycol 400 is 1:(0.5 to 3.0), and the mass ratio of diethylene glycol monoethyl ether to polyethylene glycol 3350 is 1:(0.5 to 3.0);
more preferably, the pharmaceutical composition comprises, in weight percent,
| | |
|---|---|
| active pharmaceutical ingredient | 0.1% to 2.0% |
| diethylene glycol monoethyl ether | 20.0% to 33.8% |
| polyethylene glycol 400 | 30.4% to 52.9% |
| polyethylene glycol 3350 | 25.8% to 40.0%; |
| still more preferably, | |
the pharmaceutical composition comprises, in weight percent,
| | |
|---|---|
| active pharmaceutical ingredient | 0.1 to 2.0% |
| diethylene glycol monoethyl ether | 20.0 to 33.8% |
| polyethylene glycol 400 | 30.4% to 52.9% |
| polyethylene glycol 3350 | 25.8% to 40.0% |
| butylated hydroxytoluene | 0% to 0.1% |
| methylparaben | 0% to 0.1% |
| tartrazine | 0% to 0.001% |
| beeswax | 0% to 30.65% |
| cetearyl alcohol | 0% to 9.8% |
| white petrolatum | 0% to 22.1% |
| propylene glycol | 0% to 20.0%. |

5. A topical preparation, wherein the topical preparation comprises the pharmaceutical composition according to any one of claims 1 to 4.

6. The topical preparation according to claim 5, wherein the topical preparation is a liquid preparation or semi-solid preparation;
the liquid preparation is preferably a liniment or tincture; the semi-solid preparation is preferably a cream, organogel, ointment, or paste;
preferably, the ointment is formulated to comprise, in weight percent,
| | |
|---|---|
| active pharmaceutical ingredient | 0.1% to 2.0% |
| diethylene glycol monoethyl ether | 20.0% to 33.8% |
| polyethylene glycol 400 | 30.4% to 52.9% |
| polyethylene glycol 3350 | 25.8% to 40.0%; |
more preferably,
the ointment is formulated to comprise, in weight percent,
| | |
|---|---|
| active pharmaceutical ingredient | 0.6 to 2.0% |
| diethylene glycol monoethyl ether | 20.0 to 33.8% |
| polyethylene glycol 400 | 30.4% to 52.9% |
| polyethylene glycol 3350 | 26.3% to 40.0%; |
or,
the ointment is formulated to comprise, in weight percent,
| | |
|---|---|
| active pharmaceutical ingredient | 0.1 to 1.5% |
| diethylene glycol monoethyl ether | 20.0% |
| polyethylene glycol 400 | 52.5% |
| polyethylene glycol 3350 | 25.8% to 27.2% |
| butylated hydroxytoluene | 0.1% |
| methylparaben | 0.1%; |
or,
| | |
|---|---|
| the ointment is formulated to comprise, in weight percent, | |
| active pharmaceutical ingredient | 0.1 to 1.5% |
| diethylene glycol monoethyl ether | 23.0% |
| polyethylene glycol 400 | 45.3% |
| polyethylene glycol 3350 | 30.0% to 31.4% |
| butylated hydroxytoluene | 0.1% |
| methylparaben | 0.1% |
| tartrazine | 0.00025% to 0.001%; |
or,
the ointment comprises, in weight percent,
| | |
|---|---|
| active pharmaceutical ingredient | 0.1 to 2.0% |
| diethylene glycol monoethyl ether | 20.0 to 33.8% |
| polyethylene glycol 400 | 30.4% to 52.9% |
| polyethylene glycol 3350 | 25.8% to 50.0% |
| paraffin | 0% to 5% |
| beeswax | 0% to 30.65% |
| cetearyl alcohol | 0% to 9.8% |
| white petrolatum | 0% to 22.6% |
| glyceryl monostearate | 0% to 10% |
| propylene glycol | 0% to 20.0% |
| butylated hydroxytoluene | 0% to 0.1% |
| methylparaben | 0% to 0.1% |
| polyethylene glycol 600 | 0% to 25.0% |
| light liquid paraffin | 0% to 5.0% |
| soybean oil | 0% to 5.0% |
| medium-chain triglycerides | 0% to 5.0%; |
or,
the ointment comprises, in weight percent,
| | |
|---|---|
| active pharmaceutical ingredient | 0.1 to 2.0% |
| diethylene glycol monoethyl ether | 20.0 to 33.8% |
| polyethylene glycol 400 | 30.4% to 52.9% |
| polyethylene glycol 3350 | 25.8% to 40.0% |
| butylated hydroxytoluene | 0% to 0.1% |
| methylparaben | 0% to 0.1% |
| tartrazine | 0% to 0.001% |
| beeswax | 0% to 30.65% |
| cetearyl alcohol | 0% to 9.8% |
| white petrolatum | 0% to 22.1% |
| propylene glycol | 0% to 20.0%; |
still more preferably,
the ointment is formulated to comprise, in weight percent,
| | |
|---|---|
| active pharmaceutical ingredient | 0.6% |
| diethylene glycol monoethyl ether | 20.0% |
| polyethylene glycol 400 | 52.9% |
| polyethylene glycol 3350 | 26.5%; |
or,
| | |
|---|---|
| the ointment is formulated to comprise, in weight percent, | |
| active pharmaceutical ingredient | 1.2% |
| diethylene glycol monoethyl ether | 20.0% |
| polyethylene glycol 400 | 52.5% |
| polyethylene glycol 3350 | 26.3%; |
or,
the ointment is formulated to comprise, in weight percent,
| | |
|---|---|
| active pharmaceutical ingredient | 1.2% |
| diethylene glycol monoethyl ether | 20.0% |
| polyethylene glycol 400 | 38.8% |
| polyethylene glycol 3350 | 40.0%; |
or,
the ointment is formulated to comprise, in weight percent,
| | |
|---|---|
| active pharmaceutical ingredient | 2.0% |
| diethylene glycol monoethyl ether | 33.8% |
| polyethylene glycol 400 | 30.4% |
| polyethylene glycol 3350 | 33.8%; |
or,
the ointment is formulated to comprise, in weight percent,
| | |
|---|---|
| active pharmaceutical ingredient | 0.1% |
| diethylene glycol monoethyl ether | 20.0% |
| polyethylene glycol 400 | 52.5% |
| polyethylene glycol 3350 | 27.2% |
| butylated hydroxytoluene | 0.1% |
| methylparaben | 0.1%; |
or,
the ointment is formulated to comprise, in weight percent,
| | |
|---|---|
| active pharmaceutical ingredient | 0.3% |
| diethylene glycol monoethyl ether | 20.0% |
| polyethylene glycol 400 | 52.5% |
| polyethylene glycol 3350 | 27.0% |
| butylated hydroxytoluene | 0.1% |
| methylparaben | 0.1%; |
or,
the ointment is formulated to comprise, in weight percent,
| | |
|---|---|
| active pharmaceutical ingredient | 1.0% |
| diethylene glycol monoethyl ether | 20.0% |
| polyethylene glycol 400 | 52.5% |
| polyethylene glycol 3350 | 26.3% |
| butylated hydroxytoluene | 0.1% |
| methylparaben | 0.1%; |
or,
the ointment is formulated to comprise, in weight percent,
| | |
|---|---|
| active pharmaceutical ingredient | 1.5% |
| diethylene glycol monoethyl ether | 20.0% |
| polyethylene glycol 400 | 52.5% |
| polyethylene glycol 3350 | 25.8% |
| butylated hydroxytoluene | 0.1% |
| methylparaben | 0.1%; |
or,
the ointment is formulated to comprise, in weight percent,
| | |
|---|---|
| active pharmaceutical ingredient | 0.1% |
| diethylene glycol monoethyl ether | 23.0% |
| polyethylene glycol 400 | 45.3% |
| polyethylene glycol 3350 | 31.4% |
| butylated hydroxytoluene | 0.1% |
| methylparaben | 0.1% |
| tartrazine | 0.001%; |
or,
more preferably, the ointment is formulated to comprise, in weight percent,
| | |
|---|---|
| active pharmaceutical ingredient | 0.3% |
| diethylene glycol monoethyl ether | 23.0% |
| polyethylene glycol 400 | 45.3% |
| polyethylene glycol 3350 | 31.2% |
| butylated hydroxytoluene | 0.1% |
| methylparaben | 0.1% |
| tartrazine | 0.0007%; |
more preferably, the ointment is formulated to comprise, in weight percent,
| | |
|---|---|
| active pharmaceutical ingredient | 1.0% |
| diethylene glycol monoethyl ether | 23.0% |
| polyethylene glycol 400 | 45.3% |
| polyethylene glycol 3350 | 30.5% |
| butylated hydroxytoluene | 0.1% |
| methylparaben | 0.1% |
| tartrazine | 0.00025%; |
or,
the ointment is formulated to comprise, in weight percent,
| | |
|---|---|
| active pharmaceutical ingredient | 1.5% |
| diethylene glycol monoethyl ether | 23.0% |
| polyethylene glycol 400 | 45.3% |
| polyethylene glycol 3350 | 30.0% |
| butylated hydroxytoluene | 0.1% |
| methylparaben | 0.1%. |

7. A method for preparing the pharmaceutical composition as defined in any one of claims 1 to 6, comprising the following steps:
(1) preparing an API solution by mixing an active pharmaceutical ingredient, diethylene glycol monoethyl ether, and polyethylene glycol 400 at 50°C to 80°C until dissolved;
(2) preparing a matrix solution by heating polyethylene glycol 3350 to melting at 50°C to 80°C;
(3) preparing an ointment by mixing the API solution and the matrix solution with stirring.

8. The method for preparing the pharmaceutical composition according to claim 7, wherein in step (1), a temperature at which the mixing is performed is 50°C or 80°C;
and/or, in step (2), raw materials further comprise one or more of an antioxidant, preservative, hardening agent, and moisturizing agent;
and/or, in step (2), raw materials further comprise diethylene glycol monoethyl ether; preferably, when the raw materials in step (2) comprise diethylene glycol monoethyl ether, then diethylene glycol monoethyl ether in step (1) is used in an amount that is 2/3 of a prescribed amount, and diethylene glycol monoethyl ether in step (2) is used in an amount that is 1/3 of the prescribed amount;
and/or, in step (2), a temperature at which the heating is performed is 50°C or 80°C;
and/or, in step (3), a speed at which the stirring is performed is 315 rpm;
and/or, in step (3), a duration for which the stirring is performed is 10 minutes to 15 minutes;
and/or, in step (3), a temperature at which the mixing is performed is 50°C to 60°C;
and/or, after the mixing in step (3), a cooling operation may further be included.

9. A use of the pharmaceutical composition as defined in any one of claims 1 to 4, or a topical preparation as defined in claim 5 or 6, in the manufacture of a medicament for treating an autoimmune disease;
preferably, the autoimmune disease is arthritis, rheumatoid arthritis, psoriasis, psoriasis, osteoarthritis, regional enteritis, ankylosing spondylitis, autoimmune eye disease, dry eye syndrome, atopic dermatitis, contact dermatitis, systemic lupus erythematosus, or alopecia areata.

10. A method for treating an autoimmune disease, comprising administering to a subject a therapeutically effective amount of the pharmaceutical composition as defined in any one of claims 1 to 4, or the topical preparation as defined in claim 5 or 6;
preferably, the autoimmune disease is arthritis, rheumatoid arthritis, psoriasis, psoriasis, osteoarthritis, regional enteritis, ankylosing spondylitis, autoimmune eye disease, dry eye syndrome, atopic dermatitis, contact dermatitis, systemic lupus erythematosus, or alopecia areata.
